(19) **Europäisches Patentamt / European Patent Office / Office européen des brevets**

(11) **EP 3 122 232 B1**

(12) **EUROPEAN PATENT SPECIFICATION**

(45) Date of publication and mention
of the grant of the patent:
**21.10.2020 Bulletin 2020/43**

(51) Int Cl.:
**A61B 5/055** (2006.01) **G06T 7/33** (2017.01)

(21) Application number: **15769289.8**

(86) International application number:
**PCT/US2015/023212**

(22) Date of filing: **28.03.2015**

(87) International publication number:
**WO 2015/149042 (01.10.2015 Gazette 2015/39)**

(54) **ALIGNMENT OF Q3D MODELS WITH 3D IMAGES**

AUSRICHTUNG VON Q3D-MODELLEN MIT 3D-BILDERN

ALIGNEMENT DE MODÈLES Q3D AVEC DES IMAGES 3D

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2014 US 201461971749 P**
**23.12.2014 US 201462096515 P**

(43) Date of publication of application:
**01.02.2017 Bulletin 2017/05**

(73) Proprietor: **Intuitive Surgical Operations, Inc.**
**Sunnyvale, CA 94086 (US)**

(72) Inventors:
• **PANESCU, Dorin**
**San Jose, CA 95136 (US)**

• **JONES, Daniel H.**
**Alexandria, VA 22305 (US)**

(74) Representative: **MacDougall, Alan John Shaw et al**
**Mathys & Squire LLP**
**The Shard**
**32 London Bridge Street**
**London SE1 9SG (GB)**

(56) References cited:
WO-A1-2012/059253    CA-A1- 2 859 998
US-A1- 2003 158 477    US-A1- 2007 171 369
US-A1- 2008 004 603    US-A1- 2008 058 593
US-A1- 2010 312 096    US-A1- 2012 155 731
US-A1- 2012 182 294    US-B1- 6 650 927

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

RELATED APPLICATIONS

This application claims the benefit of priority to U.S. provisional patent application no. 61/971,749, filed on March 28, 2014, and entitled "QUANTITATIVE THREE-DIMENSIONAL IMAGING OF SURGICAL SCENES"; and to U.S. provisional patent application no. 62/096,515, filed on December 23, 2014, and entitled "ALIGNMENT OF Q3D MODELS WITH 3D IMAGES".

FIELD

The invention relates in general to surgical endoscopy systems having associated image sensors, and more particularly, to determining three-dimensional coordinates of physical structures displayed in surgical images.

BACKGROUND

Quantitative three-dimensional (Q3D) vision provides numerical information about the actual physical (x, y, z) 3D coordinates of target points in a real world scene. With quantitative 3D vision, a person not only can obtain a three-dimensional perception of a real world scene, but also can obtain numerical information about physical dimensions of objects in the scene and physical distances between objects in the scene. In the past, some Q3D systems have been proposed that use time-of-flight related information or phase information to determine 3D information about a scene. Other Q3D systems have used structured light to determine 3D information about a scene.

The use of time-of-flight information is disclosed in U.S. Patent No. 6,323,942, entitled, "CMOS-compatible three-dimensional image sensor IC", which discloses a three-dimensional imaging system that includes a two-dimensional array of pixel light sensing detectors fabricated on a common IC using CMOS fabrication techniques. Each detector has an associated high speed counter that accumulates clock pulses in number directly proportional to time-of-flight (TOF) for a system-emitted pulse to reflect from an object point and be detected by a pixel detector focused upon that point. The TOF data provides a direct digital measure of distance from the particular pixel to a point on the object reflecting the emitted light pulse. In a second embodiment, the counters and high speed clock circuits are eliminated, and instead each pixel detector is provided with a charge accumulator and an electronic shutter. The shutters are opened when a light pulse is emitted and closed thereafter such that each pixel detector accumulates charge as a function of return photon energy falling upon the associated pixel detector. The amount of accumulated charge provides a direct measure of round-trip TOF.

The use of time delay information is disclosed in U.S. Patent No. 8,262,559, entitled, "Apparatus and method for endoscopic 3D data collection", which discloses a modulated measuring beam and a light-transmitting mechanism for conducting the measuring beam onto an area to be observed, where the light-transmitting mechanism includes an illuminating lens, in addition to a light-imaging mechanism for imaging a signal beam from the area to be observed at least onto a phase-sensitive image sensor. Time delays, which may correspond to differences in depth in the millimeter range, result in phase information that makes possible the production of an image that depicts depth and distance information.

The use of structured light to determine physical coordinates of objects in a visual image is disclosed in U.S. Pat. App. Pub. No. 2012/0190923, entitled "Endoscope"; and in C. Schmalz et al., "An endoscopic 3D scanner based on structured light", Medical Image Analysis, 16 (2012) 1063-1072. A triangulation method is used to measure the topography of a surface. Structured light in the form of projection rays, which may have a range of different color spectra, are incident upon and are reflected from a surface. The reflected rays are observed by a camera that is calibrated to use the reflected color spectra information to determine 3D coordinates of the surface. More specifically, the use of structured light typically involves shining a light pattern on a 3D surface, and determining physical distances based upon a deformation pattern of the light due to contours of the physical object.

An imager array camera has been built that includes a plurality of pixel arrays that can be used to compute scene depth information for pixels in the array. High resolution (HR) images are generated from multiple low resolution (LR) images. A reference viewpoint is selected and an HR image is generated as seen by that viewpoint. A parallax processing technique utilizes the effects of aliasing to determine pixel correspondences for non-reference images with respect to the reference image pixels. Fusion and superresolution are utilized to produce the HR image from the multiple LR images. See e.g., U.S. Patent No. 8,514,491, entitled "Capturing and Processing Images using Monolithic Camera Array with Heterogeneous Imager"; U.S. Pat. App. Pub. No. 2013/0070060, entitled, "Systems and Methods for Determining Depth from multiple Views of a Scene that Include Aliasing using Hypothesized Fusion"; and K. Venkataraman et al., "PiCam: An ultra-Thin high Performance Monolithic Camera Array".

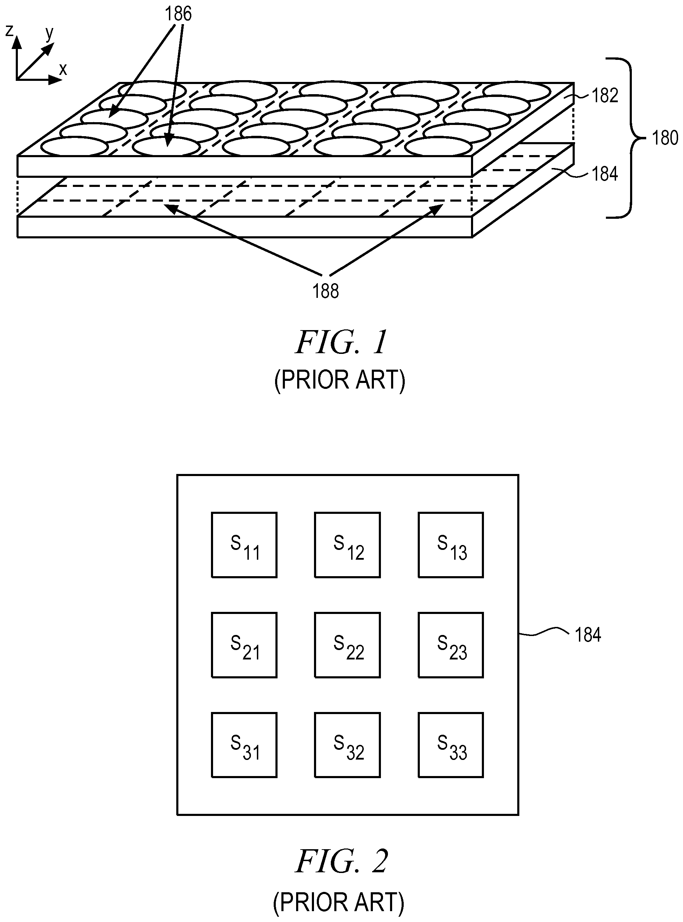

Figure 1 is an illustrative drawing showing details of a known imager sensor 180 in accordance with some embodiments. The image sensor 180 includes an arrangement of sensors 184. Each sensor in the arrangement includes a two dimensional arrangement of pixels having at least two pixels in each dimension. Each sensor includes a lens stack 186. Each lens stack 186 has a corresponding focal plane 188. Each lens stack 186 creates a separate optical channel that resolves an image onto a corresponding arrangement of pixels disposed in its corresponding focal 188 plane. The pixels act as light sensors, and each focal plane 188 with its multiple pixels acts as an image sensor. Each sensor with its focal plane 188 occupies a region of the sensor arrangement different from regions of the sensor arrangement occupied by other sensors and focal planes.

Figure 2 is an illustrative drawing showing a simplified plan view of the known arrangement of sensors 184 of Figure 1 that includes sensors labeled as sensors $S_{11}$ through $S_{33}$. The imager sensor arrangement 184 is fabricated on a semiconductor chip to include a plurality of sensors $S_{11}$ through $S_{33}$. Each of the sensors $S_{11}$ through $S_{33}$ includes a plurality of pixels (e.g., 0.32 megapixels) and is coupled to peripheral circuitry (not shown) that includes independent read-out control and pixel digitization. In some embodiments, the sensors $S_{11}$ through $S_{33}$ are arranged into a grid format as illustrated in Figure 2. In other embodiments, the sensors are arranged in a non-grid format. For example, the sensors may be arranged in a circular pattern, zigzagged pattern, scattered pattern, or irregular pattern including sub-pixel offsets.

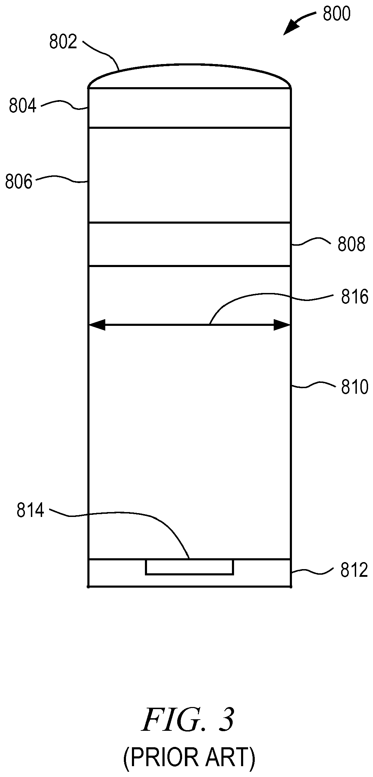

Each individual pixel of the sensors 184 of Figures 1-2 includes a microlens pixel stack. Figure 3 is an illustrative drawing of a known microlens pixel stack of the sensors of Figures 1-2. The pixel stack 800 includes a microlens 802, which is positioned above an oxide layer 804. Typically beneath the oxide layer 804 there may be a color filter 806, which is disposed above a nitride layer 808, which is disposed above a second oxide layer 810, which sits atop a silicon layer 812 that includes the active area 814 (typically a photodiode) of the individual pixel. The primary role of the microlens 802 is to gather the light incident on its surface and to focus that light onto the small active area 814. The pixel aperture 816 is determined by the spread of the microlens.

Additional information concerning the above-described known imager sensor arrangement architecture is provided in U.S. Patent No. U.S. 8,514,491 B2 (filed Nov. 22, 2010), and in U.S. Patent Application Pub. No. US 2013/0070060 A1 (filed Sep. 19, 2012).

US 6 650 927 discloses a method and apparatus for mapping a structure in a body of a subject which includes capturing a three-dimensional (3D) image of the structure comprising diagnostic information, and generating a 3D geometrical map of the structure using a probe inserted into the structure. The image is registered with the map, such that each of a plurality of image points in the image is identified with a corresponding map point in the map. The map is displayed such that the diagnostic information associated with each of the image points is displayed at the corresponding map point.

US 2015/049167 discloses a photographic device including a camera array in which plural photographic units are arrayed on a leading end of an arm formed to be capable of passing through a tubular body which is inserted into a body from outside the body. The camera array is displaceably disposed between an alignment location whereat the plural photographic units are arrayed along an extension of the arm and a deployment location whereat the plural photographic units are arrayed laterally to the arm.

SUMMARY

The present invention provides a system and a method for producing an image of a surgical scene as defined in the appended claims.

In one example, systems and methods are provided to align a Q3D model of a surface of a three-dimensional (3D) structure with a 3D visual representation of same 3D structure, representation which includes sub-surface detail. The system comprises, at least, a Q3D endoscope, at least one processor, or processing subsystem, which generates the Q3D model and performs the alignment, an input to receive the 3D visual representation and an output to store, display, or further process or manipulate the result of the alignment of the Q3D model and the 3D visual representation.

In some embodiments, the 3D visual representation may include 3D MRI or CT images of the 3D anatomic structure. Multiple fiducial points indicative of surface contours of the 3D structure are identified within the 3D visual representation. In addition, multiple fiducial points indicative of the same surface contours of the 3D structure are identified within the Q3D model. One or more geometric transformations are applied to the 3D visual representation of the 3D structure to align the identified fiducial points in the 3D visual representation with the identified fiducial points in the Q3D model. A visual image representation of the 3D structure is produced that includes a view of a surface of the 3D structure that is within the field of view of the Q3D endoscope and that also includes a view of the internal sub-surface target structure.

BRIEF DESCRIPTION OF THE DRAWINGS

Aspects of the present disclosure are best understood from the following detailed description when read with the accompanying figures. It is emphasized that, in accordance with the standard practice in the industry, various features are not drawn to scale. In fact, the dimensions of the various features may be arbitrarily increased or reduced for clarity of discussion. In addition, the present disclosure may repeat reference numerals and/or letters in the various examples. This repetition is for the purpose of simplicity and clarity and does not in itself dictate a relationship between the various embodiments and/or configurations discussed.

Figure 1 is an illustrative drawing showing details of a known imager sensor.

Figure 2 is an illustrative drawing showing a simplified plan view of a known sensor array of the imager sensor of Figure 1.

Figure 3 is an illustrative drawing of a known microlens pixel stack of a pixel within a sensor of the sensor array of Figure 2.

Figure 4 is an illustrative drawing showing a perspective view of a surgical scene through a viewer in accordance with some embodiments.

Figure 5 is an illustrative block diagram of a teleoperation surgery system to perform minimally invasive surgical procedures using one or more mechanical arms in accordance with some embodiments.

Figure 6 is an illustrative perspective view of a patient-side system of the system of Figure 5 in accordance with some embodiments.

Figure 7A is an illustrative drawing of a first endoscope that includes a first image capture system in accordance with some embodiments.

Figure 7B is an illustrative drawing of a second endoscope that includes a second image capture system in accordance with some embodiments.

Figure 8 is illustrative block diagram showing control blocks associated with the first endoscope that includes the first image capture system of Figure 7A and showing the system in operation, in accordance with some embodiments.

Figure 9 is an illustrative flow diagram representing a process to determine a quantitative three dimensional location of a physical target in accordance with some embodiments.

Figure 10 is an illustrative flow diagram showing certain details of a process generally corresponding to module Figure 9 to systematically select targets in accordance with some embodiments.

Figure 11 is an illustrative drawing of an example sensor imager array that includes multiple sensors and that is disposed to have a field of view that encompasses an illustrative three dimensional physical world scene that includes three illustrative objects in accordance with some embodiments.

Figure 12 is an illustrative drawing representing projections of the multiple physical objects of Figure 11 onto multiple sensors in accordance with some embodiments.

Figure 13 is an illustrative drawing indicating selection of a region of interest from within a real-world scene in accordance with some embodiments.

Figure 14 is an illustrative drawing showing detail as to relative geometric offset of the projected images in sensors multiple sensors in accordance with some embodiments.

Figure 15 is an illustrative drawing showing the projected images in certain example sensors within the region of interest (ROI) shifted to the right to align with the projected images in a designated reference sensor within the ROI in accordance with some embodiments.

Figure 16 is an illustrative drawing showing projections of a selected target point onto multiple sensors in accordance with some embodiments.

Figure 17 is an illustrative drawing showing a portion of an imager array that includes the multiple sensors of Figure 16 and the selected target point T disposed at location in physical space in accordance with some embodiments.

Figure 18 is an illustrative elevation view of the projection of the currently selected target point T onto the multiple image sensors of Figure 16 in accordance with some embodiments.

Figure 19 is an illustrative drawing showing the disposition of a currently selected target relative to the multiple sensors as described above with reference to Figure 17 and also showing y-direction pixel offsets for the candidate pixel in each of the sensors in accordance with some embodiments.

Figure 20 is an illustrative flow diagram representing a first process to use Q3D information during a surgical procedure in accordance with some embodiments.

Figures 21 is an illustrative drawing showing menu selections displayed on a display screen in accordance with the process of Figure 20 in accordance with some embodiments.

Figures 22A-22B are illustrative drawings representing certain details of receiving user input in accordance with the process of Figure 20 in accordance with some embodiments.

Figure 23 is an illustrative flow diagram representing a second process to use Q3D information during a surgical procedure in accordance with some embodiments.

Figures 24 is an illustrative drawing showing menu selections displayed on a display screen in accordance with the process of Figure 23 in accordance with some embodiments.

Figure 25 is an illustrative drawing showing details of a processor that displays a Q3D model in a 3D perspective on a 3D display in accordance with some embodiments.

Figure 26 is an illustrative drawing representing an example of a 3D volumetric image representation of an example anatomical structure.

Figure 27A is an illustrative drawing showing a cross section of a 3D MRI/CT representation that shows surface structures of an anatomical structure and sub-surface structures of the anatomical structure.

Figure 27B is an illustrative drawing representing a 3D view of an anatomical object and associated 2D image slices through the object created using CT or MRI techniques.

Figure 28 is an illustrative drawing showing a Q3D endoscope positioned to capture image information corresponding to a surface portion of the anatomical structure of Figure 26 in accordance with some embodiments.

Figure 29 is an illustrative drawing conceptually representing a process of aligning the example 3D MRI/CT cross section of the anatomical structure of Figure 26 with the example Q3D model of Figure 28 in accordance with some embodiments.

Figure 30 is an illustrative flow diagram showing details of a process to align a Q3D model with a 3D MRI/CT representation in accordance with some embodiments.

Figure 31 is an illustrative flow diagram representing a process to generate a visual display based upon a Q3D-MRI/CT combination model in accordance with some embodiments.

Figure 32 is an illustrative drawing representing a Q3D-MRI/CT combination model in accordance with some embodiments.

Figures 33A-33C are illustrative drawings representing three different Q3D-MRI/CT combination models based upon three different surface regions of the anatomical structure being within the field of view of the endoscope in accordance with some embodiments.

DESCRIPTION OF EMBODIMENTS

The following description is presented to enable any person skilled in the art to create and use a surgical endoscopy system having multiple image sensors, each sensor including a pixel array that is separate from pixel arrays of other sensors, so as to determine three-dimensional coordinates of physical structures within a field of view of the image sensors. Various modifications to the embodiments will be readily apparent to those skilled in the art, and the generic principles defined herein may be applied to other embodiments and applications without departing from the scope of the inventive subject matter. Moreover, in the following description, numerous details are set forth for the purpose of explanation. However, one of ordinary skill in the art will realize that the inventive subject matter might be practiced without the use of these specific details. In other instances, well-known machine components, processes and data structures are shown in block diagram form in order not to obscure the disclosure with unnecessary detail. Identical reference numerals may be used to represent different views of the same item in different drawings. Flow diagrams in drawings referenced below are used to represent processes. A computer system may be configured to perform some of these processes. Modules within flow diagrams representing computer implemented processes represent the configuration of a computer system according to computer program code to perform the acts described with reference to these modules. Thus, the inventive subject matter is not intended to be limited to the embodiments shown, but the invention is defined in the appended claims. Any "aspect", "example" or "embodiment" of the description not falling within the scope of the claims does not form part of the invention and is provided for illustrative purposes only.

Brief Overview

In accordance with some embodiments, an imager that includes that includes a sensor array is associated with an endoscope. The image sensor array includes multiple sensors, and each sensor includes an array of pixels. A portion of the endoscope is inserted into a human body cavity, and a target object in a field of view of the image sensor array is illuminated using a light source. A physical location and/or dimensions of the target object is determined based upon images of the target object projected onto individual sensors of the array.

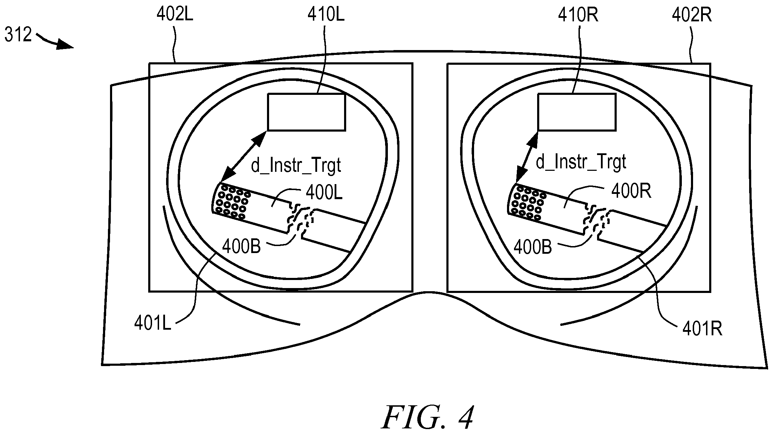

Figure 4 is an illustrative drawing showing a perspective view of a surgical scene through a viewer 312 in accordance with some embodiments. A viewing system having two viewing elements 401R, 401L can provide a good 3D viewing perspective. Numerical values representing physical dimension and/or location information for physical structures in the surgical scene are shown overlaid onto the surgical scene image. For example, a numerical distance value "d_Instr_Trgt" is shown displayed within the scene between instrument 400 and target 410.

Teleoperation Medical System

Teleoperation refers to operation of a machine at a distance. In a minimally invasive teleoperation medical system, a surgeon may use an endoscope that includes a camera to view a surgical site within a patient's body. Stereoscopic images have been captured, which allow the perception of depth during a surgical procedure. A camera system, which is mounted on an endoscope and which includes an imager sensor array, provides quantitative three-dimensional information plus color and illumination data that can be used to generate three-dimensional images in accordance with some embodiments.

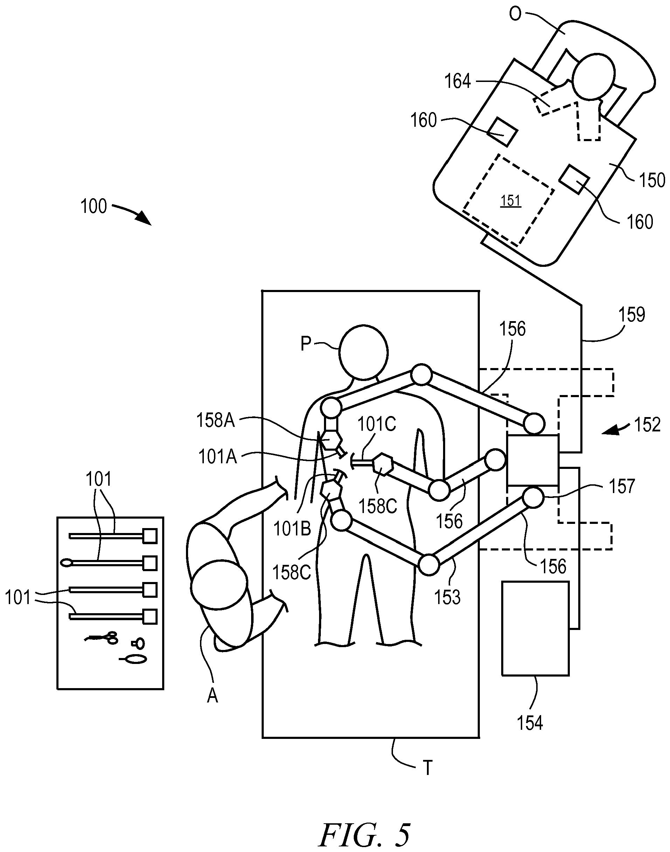

Figure 5 is an illustrative block diagram of a teleoperation surgery system 100 to perform minimally invasive surgical procedures using one or more mechanical arms 158 in accordance with some embodiments. Aspects of system 100 includes telerobotic and autonomously operating features. These mechanical arms often support an instrument. For instance, a mechanical surgical arm (e.g., the center mechanical surgical arm 158C) may be used to support an endoscope with a stereo or three-dimensional surgical image capture device 101C, such as an endoscope associated a Q3D image sensor array. The mechanical surgical arm 158C may include a sterile adapter, or a clamp, clip, screw, slot/groove, or other fastener mechanism to mechanically secure an endoscope that includes the image capture device 101C to the mechanical arm. Conversely, the endoscope with image capture device 101C may include physical contours and/or structures complementary to those of the mechanical surgical arm 158C so as to securely interfit with them.

A user or operator O (generally a surgeon) performs a minimally invasive surgical procedure on patient P by manipulating control input devices 160 at a master control console 150. The operator can view video frames of images of a surgical site inside a patient's body through a stereo display device 164, which includes the viewer 312 described above with reference to Figure 4. A computer 151 of the console 150 directs movement of teleoperationally controlled endoscopic surgical instruments 101A-101C via control lines 159, effecting movement of the instruments using a patient-side system 152 (also referred to as a patient-side cart).

The patient-side system 152 includes one or more mechanical arms 158. Typically, the patient-side system 152 includes at least three mechanical surgical arms 158A-158C (generally referred to as mechanical surgical arms 158) supported by corresponding positioning set-up arms 156. The central mechanical surgical arm 158C may support an endoscopic camera 101C suitable for capture of Q3D information for images within a field of view of the camera. The mechanical surgical arms 158A and 158B to the left and right of center may support instruments 101A and 101B, respectively, which may manipulate tissue.

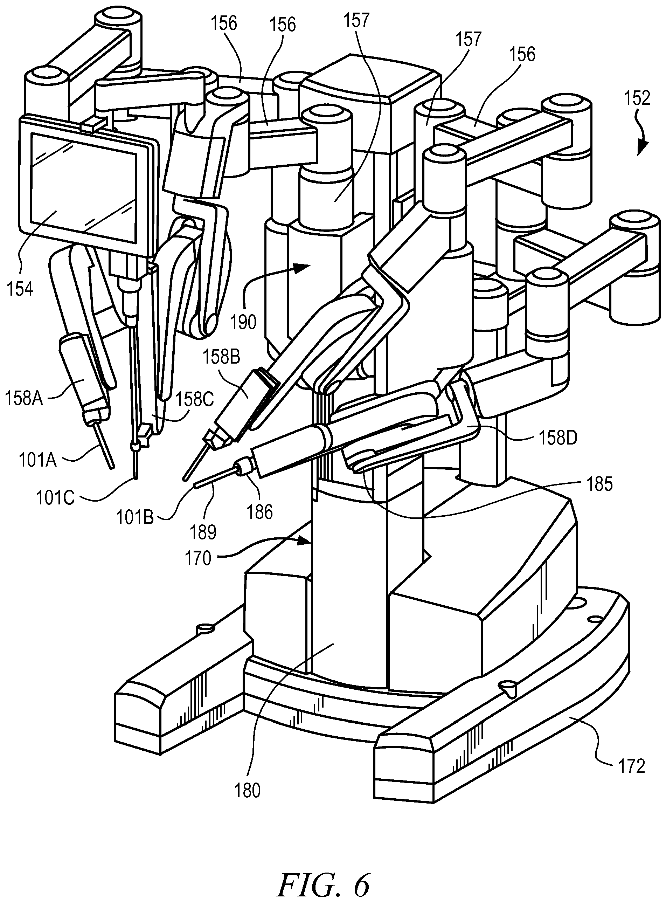

Figure 6 is an illustrative perspective view of the patient-side system 152 in accordance with some embodiments. The patient-side system 152 comprises a cart column 170 supported by a base 172. One or more mechanical insertion surgical arms/links 158 are respectively attached to one or more set-up arms 156 that are a part of the positioning portion of the patient-side system 152. Situated approximately at a central location on base 172, the cart column 170 includes a protective cover 180 that protects components of a counterbalance subsystem and a braking subsystem from contaminants.

Excluding a monitor arm 154, each mechanical surgical arm 158 is used to control instruments 101A-101C. Moreover, each mechanical surgical arm 158 is coupled to a set-up arm 156 that is in turn coupled to a carriage housing 190 in one embodiment of the invention. The one or more mechanical surgical arms 158 are each supported by their respective set-up arm 156, as is illustrated in Figure 6.

The mechanical surgical arms 158A-158D may each include one or more displacement transducers, orientational sensors, and/or positional sensors 185 to generate raw uncorrected kinematics information to assist in initial acquisition by a tracking system and tracking of instruments. The instruments may also include a displacement transducer, a positional sensor, and/or orientation sensor 186 in some embodiments of the invention. Moreover, one or more instruments may include a marker 189 to assist in acquisition and tracking of the instruments.

Additional information about a teleoperation medical system is provided in U.S. Patent Application Pub. No. US 2012/0020547, (filed Sep. 30, 2011).

Endoscopic Imager System

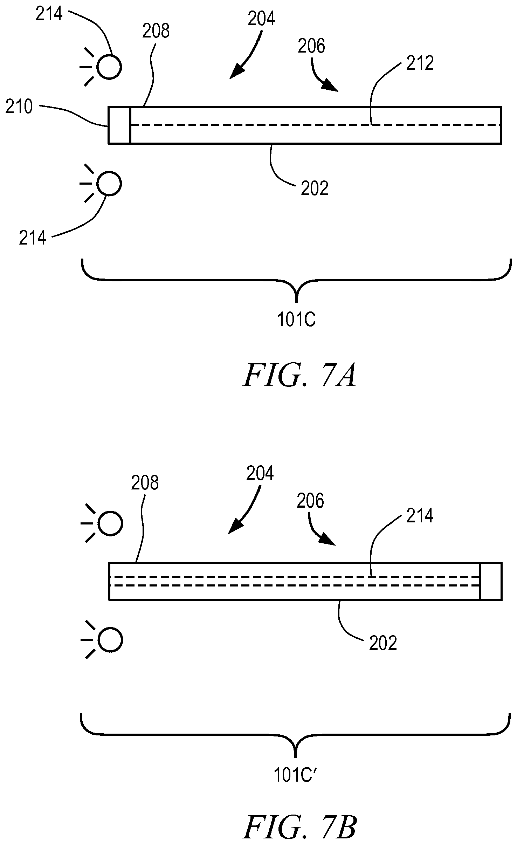

Figure 7A is an illustrative drawing of a first endoscope with a first image capture system 101C in accordance with some embodiments. The image capture system 101C includes an endoscope that includes elongated portion 202, which includes a first end portion 204 and a second end portion 206 and a tip portion 208 of the first end portion 204. The first end portion 204 is dimensioned to be inserted into a human body cavity. A sensor array 210, which includes multiple image sensors (not shown), is coupled at the tip portion 208 of the first end portion 204. In accordance with some embodiments, each sensor in the sensor array 210 includes an array of pixels. The elongated portion 202 has a length sufficient to position the tip portion 208 close enough to a target object within the body cavity so that the object can be imaged by the imager sensor array 210. In accordance with some embodiments, the second end portion 206 may include physical contours and/or structures (not shown), as generally described above, so as to securely interfit with a mechanical arm (not shown). The elongated portion 202 also includes one or more electronic signal paths 212 to electronically communicate information with the imager sensor array 210. A light source 214 is disposed to illuminate the object to be imaged. In accordance with some embodiments, the light source 214 can be unstructured light, white light, color filtered light, or light at some selected wavelength, for example. In accordance with some embodiments the light source 214 is located at tip 208, and in other embodiments it is optionally located separately from endoscope 101C.

Figure 7B is an illustrative drawing of a second endoscope with a second image capture system 101C2, in accordance with some embodiments. Aspects of the second image capture system 101C2 that are essentially the same as those of the first endoscope with the first image capture system 101C are indicated by identical reference numerals and are not described again. An input to a light pipe input, such as a rod lens, is disposed at the tip portion 208 of the first end portion 204. A light pipe body extends within the elongate portion 202 so as to transmit an image received as the light pipe input to the imager sensor array 210, which is physically displaced from the tip portion 208. In some embodiments, the imager sensor array 210 is displaced far enough from the tip portion 208 so that the imager sensor array 210 is located outside the body cavity during observation of objects within the cavity.

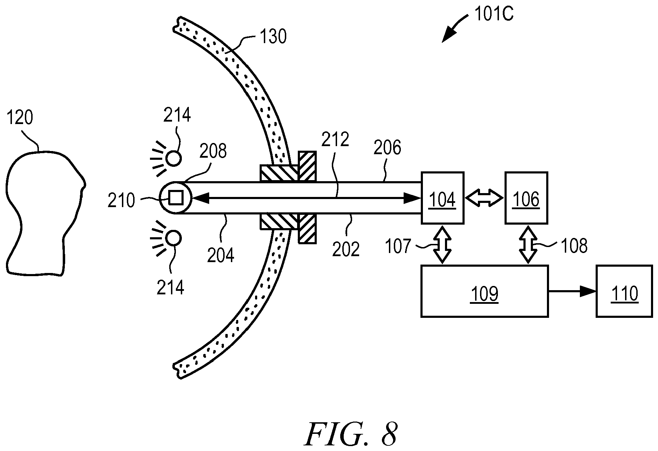

Figure 8 is illustrative block diagram showing control blocks associated with the first endoscope 101C with the first image capture system 101C of Figure 7A and showing the system in operation, in accordance with some embodiments. Images captured by the imager sensor array 210 are sent over a data bus 212 to a video processor 104, which communicates via bus 105 with a controller 106. The video processor 104 may comprise a camera control unit (CCU) and a video signal detector (VSD) board. The CCU programs or controls various settings of the imaging sensor 210, such as brightness, color scheme, white balance, etc. The VSD processes the video signal received from the imaging sensor. Alternatively, the CCU and VSD are integrated into one functional block.

In accordance with some embodiments a processor system that includes one or more than one processor is configured to perform processor functions. In some embodiments the processor system includes multiple processors configured to operate together to perform the processor functions described herein. Thus, reference herein to at least one processor configured to perform one or more functions includes a processor system in which the functions may be performed by one processor alone or by multiple processors working together.

In one implementation, the controller 106, which includes a processor and a storage device (not shown) computes the physical quantitative 3D coordinates of the points in a scene adjacent the tip 208 of the elongate portion 202 and drives both the video processor 104 and a 3D display driver 109 to compose 3D scenes, which then can be displayed on a 3D display 110. In accordance with some embodiments, Q3D information about a surgical scene is generated, such as numerical indicia of dimensions of surface contours of objects in a scene or distances from objects within the surgical scene, for example. As explained more fully below, the numerical Q3D depth information can be used to annotate a stereoscopic image of a surgical scene with distance information or surface contour information.

Data buses 107 and 108 exchange information and control signals among the video processor 104, the controller 106, and the display driver 109. In some embodiments, these elements can be integrated with the image sensor array 210 inside the body of the endoscope. Alternatively, they can be distributed internally and/or externally to the endoscope. The endoscope is shown positioned, via a cannula 140, to penetrate body tissue 130 in order to provide visualize access to a surgical scene that includes a target 120. Alternatively, the endoscope and one or more instruments may also pass through a single opening-a single incision or natural orifice-to reach a surgical site. The target 120 can be an anatomic target, another surgical instrument, or any other aspect of the surgical scene inside a patient's body.

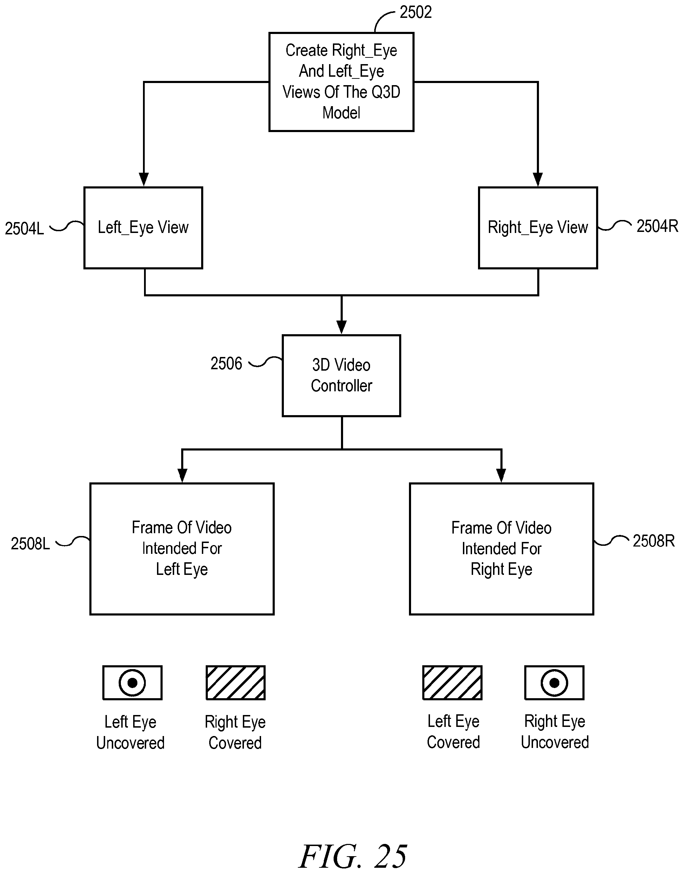

An input system 112 receives the 3D visual representation and provides it to processor 106. The input system 112 may include a storage device coupled to an electronic communication bus (not shown) that receives a 3D model such as a CRT or MRI from a system (not shown) that generates the 3D model. Processor 106, for example, can be used to compute the alignment intended between the Q3D model and the 3D visual representation. More particularly, without limitation, input system 112 may include a processor configured to establish an Ethernet communication connection between system 152 and an imaging system (not shown), such as a MRI, CT or ultrasound imaging system. Other imaging systems may be used. Other types of communication connections may be used, such as Bluetooth, WiFi, optical, etc. Alternatively, system 152 and the imaging system may be integrated in one larger system. The result of the alignment process may be saved in the storage device associated with processor 106, provided for further manipulation to external devices or system or displayed as shown in Figure 25.

Example of Q3D Information Added to an Image of a Scene

Referring once again to Figure 4 is an illustrative drawing showing a perspective view of a viewer 312 of the master control console 150 of Figure 5 in accordance with some embodiments. In accordance with some embodiments, to provide a three-dimensional perspective, the viewer 312 includes stereo images for each eye. As shown, a left image 400L and a right image 400R of the surgical site include any instruments 400 and a target 410 respectively in a left viewfinder 401L and a right viewfinder 401R. The images 400L and 400R in the viewfinders may be provided by a left display device 402L and a right display device 402R, respectively. The display devices 402L,402R may optionally be pairs of cathode ray tube (CRT) monitors, liquid crystal displays (LCDs), or other type of image display devices (e.g., plasma, digital light projection, etc.). In the preferred embodiment of the invention, the images are provided in color by a pair of color display devices 402L, 402R; such as color CRTs or color LCDs. To support backward compatibility with existing devices, stereoscopic display devices 402L and 402R may be used with a Q3D system. Alternatively, the Q3D imaging system can be connected to 3D monitors, 3D TVs, or to autostereoscopic displays, such as a display that does not require use of 3D effect eye glasses.

A viewing system having two viewing elements 401R, 401L can provide a good 3D viewing perspective. The Q3D imaging system supplements this viewing perspective with physical dimension information for physical structures in the surgical scene. The stereo viewer 312 used in conjunction with a Q3D endoscopy system, can display Q3D information overlayed onto the stereo image of the surgical scene. For example, as shown in Figure 4, the numerical Q3D distance value "d_Instr_Trgt" between instrument 400 and target 410 can be displayed within stereo viewer 312.

An explanation of a video stereo viewing system that can be used to overlay physical location and dimension information onto a 3D perspective of a surgical scene is provided in U.S. Patent Application Pub. No. US 2012/0020547, (filed Sep. 30, 2011), paragraphs [0043]-[0053] and corresponding drawings.

Processing Quantitative Three-Dimensional Physical Information

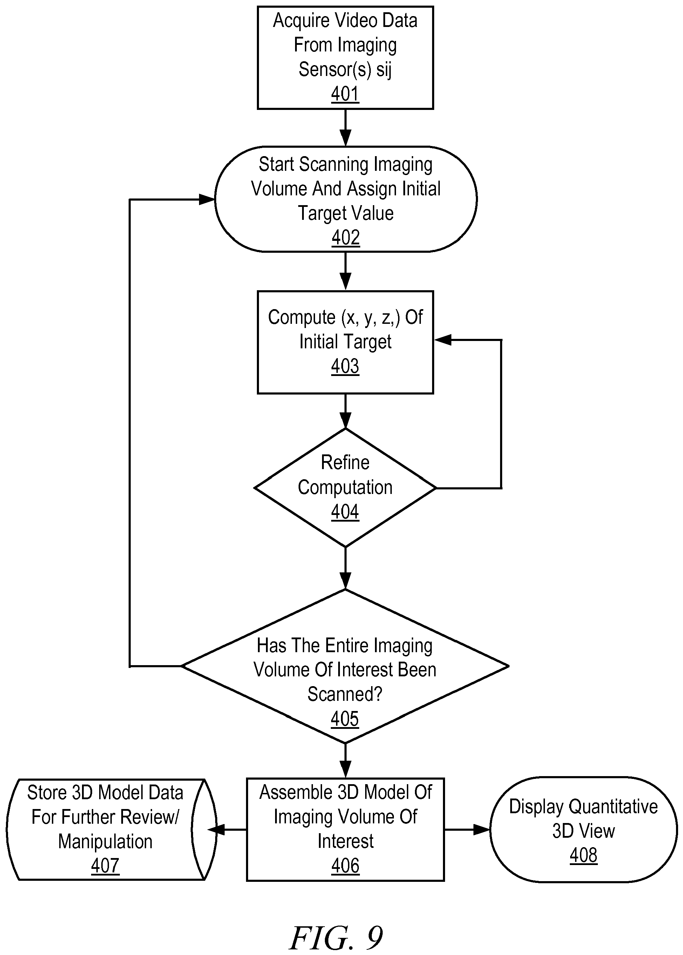

Figure 9 is an illustrative flow diagram representing a process to determine a quantitative three-dimensional location of a physical target in accordance with some embodiments. The process is described with reference to the endoscope with image capture system 101C of the embodiment of Figure 8. Module 401 configures the controller 106 to acquire video data from imaging sensors $S_{ij}$. It will be appreciated that although the image sensor array 210 "images" an entire field of view, different sensors and different pixels within different sensors in image sensor array 210 may be illuminated by image projections from different object points within the field of view. The video data, for example, may include color and light intensity data. Each pixel of each sensor may provide one or more signals indicative of the color and intensity of an image projected onto it. Module 402 configures the controller to systematically select targets from a selected region of interest in a physical world view. Module 403 configures the controller to commence the computation of the target 3D coordinates (x, y, z) with an initial ($x_0$, $y_0$, $z_0$) set. The algorithm then checks the coordinates for consistency, by using image diversity data from all sensors $S_{ij}$ that receive a projected image of the target. The coordinate computation is refined at decision module 404 until an acceptable accuracy is reached. Decision module 404 also configures the controller to determine whether the currently computed physical location is sufficiently accurate. In response to a determination that the currently computed location is not accurate enough, control flows back to module 403 to try a different possible physical location. In response to a determination that the currently computed location is sufficiently accurate, module 405 configures the controller to determine whether the entire region of interest has been scanned. In response to a determination that the entire region of interest has not been scanned, control flows back to module 402 and a different target is selected. In response to a determination that the entire region of interest has been scanned, control flows to module 406, which configures the controller to assemble a three-dimensional model of the imaging volume of interest. Assembly of a 3D image of a target based upon three-dimensional information indicating the physical position of structures of the target is known to persons of ordinary skill in the art and need not be described herein. Module 407 configures the controller to store the 3D model developed using the physical position information determined for multiple targets for further review and manipulation. For example, the 3D model could be used at a later time for surgical applications, such as sizing an implant for the particular dimensions of a patient's organ. In yet a different example, when a new surgical instrument 101 is installed on the robotic system 152, it may be necessary to call back the 3D model and display it on display 110 in order to reference the new instrument to the previous surgical scene. Module 407 may also store the result of the alignment between the 3D visual representation and the Q3D model. Module 408 configures the controller to use the physical position information determined for multiple targets to display a quantitative 3D view. An example of a Q3D view is the distance value "d_Instr_Trgt" shown in Figure 4.

It is noted that a stereoscopic display creates the illusion of viewing in three dimensions. However, an actual 3D display presents a 3D image, such as a holographic image or an image projected onto a curved surface. Typically, a 3D display allows the view to move to change viewing perspective.

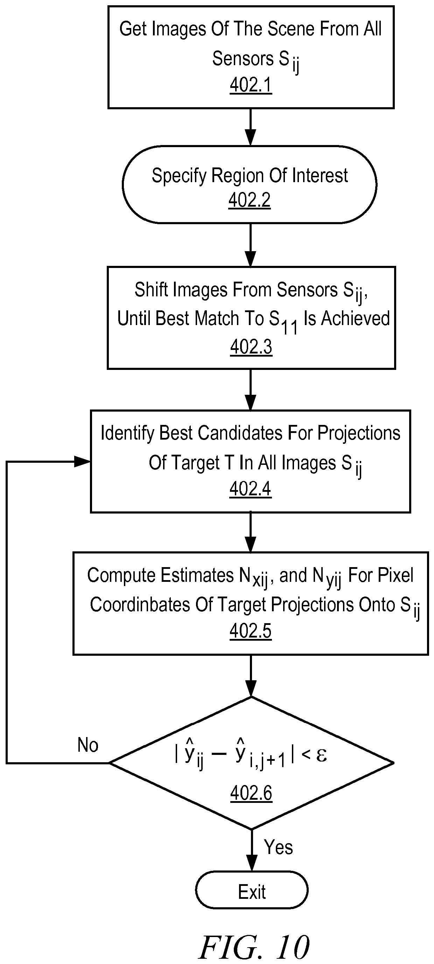

Figure 10 is an illustrative flow diagram showing certain details of a process generally corresponding to module 402 of Figure 9 in accordance with some embodiments. Module 402.1 configures the controller to capture images of a physical world scene from all sensors in the sensor array 210. Module 402.2 configures the controller to specify a region of interest from within the captured scene. Module 402.3 configures the controller to search for a best match as between scene images within the region of interest so as to identify pixel locations in different sensors that are illuminated by projections of the same target. As explained later, the best matching may be achieved, without limitation, by shifting the individual images from sensors Sij until maximizing two-dimensional cross-correlation function between the shifted image and a reference image. The reference image, for example, may be the scene image received from sensor $S_{11}$. Module 402.4 configures the controller to identify candidate pixels illuminated by projections from the same target. Module 402.5 configures the controller to compute two or more pixel coordinates ($N_x$, $N_y$) coordinates for the selected target to determine whether the candidate pixels are illuminated by a projection from the same target. Decision module 402.6 determines whether the computed 2D pixel coordinate values indicate that the candidate pixels are illuminated by a projection from the same target. The image diversity caused by viewing the same scene with multiple sensors Sij plays a role in correctly identifying ($N_x$, $N_y$) associated with a specific target in the various individual images $S_{ij}$. For example, in accordance with some embodiments, assuming a simplified scenario where only three sensors are used, $S_{11}$, $S_{12}$ and $S_{13}$, if the triplet of 2D pixel coordinates [($Nx_{11}$, $Ny_{11}$), ($Nx_{12}$, $Ny_{12}$), ($Nx_{13}$, $Ny_{13}$)] are not corresponding to projections of the same target onto [$S_{11}$, $S_{12}$ and $S_{13}$] then the quantities $\hat{y}_{12}$ and $\hat{y}_{13}$ (which are estimates of the projection shift in the y direction) will yield different values. According the equations presented later, $\hat{y}_{12}$ and $\hat{y}_{13}$ should be equal if pixel coordinates ($Nx_{11}$, $Ny_{11}$), ($Nx_{12}$, $Ny_{12}$), ($Nx_{13}$, $Ny_{13}$) come from projections of the same target.

$$\hat{y}_{12} = \frac{Ny_{11}}{Ny_{11} - Ny_{12}} \qquad (402.5 - 1)$$

$$\hat{y}_{13} = 2 \cdot \frac{Ny_{11}}{Ny_{11} - Ny_{13}} \qquad (402.5 - 2)$$

If $\hat{y}_{12}$ and $\hat{y}_{13}$ are not approximately equal then control flows back to module 402.4 and to refine the best candidates for target projections onto sensor planes $S_{ij}$. As mentioned, the above is just a simplified implementation of the algorithm. In general, as shown in Figure 10 module 402.6, the norm of the difference between $\hat{y}_{i,j}$ and $\hat{y}_{i,j+1}$ should be less than an acceptable tolerance $\varepsilon$ in order for module 402 to complete its iterations. A similar restriction should be met for the corresponding estimates for the x axis, $x_{i,j}$ and $x_{i,j+1}$. In response to a determination that the computed 2D pixel coordinate values ($N_x$, $N_y$) do indicate that the candidate pixels are illuminated by a projection from the same target, then control flows to module 403.

It will be appreciated that each pixel directly captures color and intensity information from a world scene. Moreover, in accordance with the above process, each pixel is associated with the (x, y, z) coordinates of the physical object in the world view that is projected onto the pixel. Thus, color information, illumination intensity information and physical location information, i.e. the location of the physical object that projected the color and illumination, can be associated with a pixel in a non-transitory computer readable storage device. The following Table 1 illustrates this association.

Table 1

| Pixel Identifier | Color Value | Intensity Value | Location (x, y, z) |
|---|---|---|---|

Examples of Determining Q3D Information

Example of Projection Matching

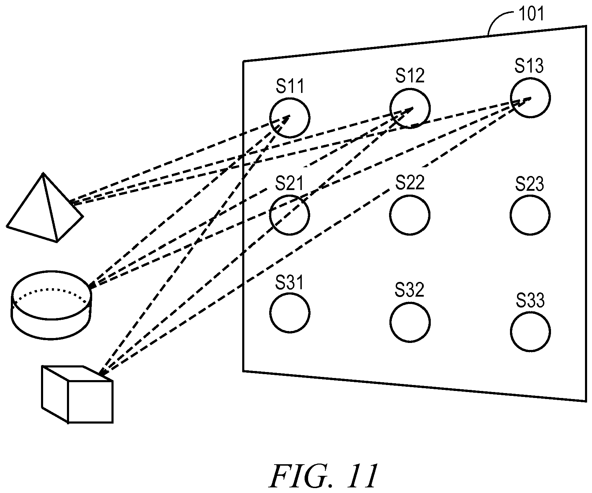

Figure 11 is an illustrative drawing of an example sensor array 210 that includes an array of sensors $S_{11}$-$S_{33}$ that is disposed to have a field of view that encompasses an illustrative three-dimensional physical world scene that includes three illustrative objects in accordance with some embodiments. Each sensor $S_{ij}$ in the array includes a two-dimensional arrangement of pixels having at least two pixels in each dimension. Each sensor includes a lens stack that creates a separate optical channel that resolves an image onto a corresponding arrangement of pixels disposed in a focal plane of the lens stack. Each pixel acts as a light sensor, and each focal plane with its multiple pixels acts as an image sensor. Each sensor $S_{11}$-$S_{33}$ with its focal plane occupies a region of the sensor array different from regions of the sensor array occupied by other sensors and focal planes. Suitable known image sensor arrays are disclosed in U.S. Patent No. U.S. 8,514, 491 (filed Nov 22, 2010) and in U.S. Patent Application Pub. No. U.S. 2013/0070060 (filed Sep 19, 2012), which are described above.

In accordance with some embodiments, the sensors are characterized by a $N_x$ and $N_y$, their total number of pixels in the x and y directions, and by field of view angles, $\theta_x$ and $\theta_y$. In some embodiments, the sensor characteristics for the x and y axes are expected to be the same. However, in alternative embodiments, the sensors have asymmetric x and y axis characteristics. Similarly, in some embodiments, all sensors will have the same total number of pixels and the same field of view angle. The sensors are distributed across the sensor array 210 in a well-controlled manner. For example, the sensors may be at $\delta$ distance apart on the two-dimensional grid shown. The sensor placement pitch $\delta$ may be symmetric or asymmetric across such grid.

In the embodiment shown in Figure 11, the sensors are arranged in a rectangular grid in which sensors $S_{11}$-$S_{13}$ occupy a top row, sensors $S_{21}$-$S_{23}$ occupy a middle row, and sensors $S_{31}$-$S_{33}$ occupy a bottom row. Each sensor includes N rows of pixels and N columns of pixels. Light rays, indicated by dashed lines, produced by a light source are reflected from each of a triangular-shaped first object, a spherical-shaped second object, and a rectangular-shaped third object, to each sensor of the imager array. For illustration purposes, only rays to sensors $S_{11}$, $S_{12}$ and $S_{13}$ in the top row are shown. The light source may be non-structured white light or ambient light, for example. Alternatively, the light source may provide light at a selected wavelength, such as in the visible or infrared spectrums, or the light may be filtered or split to provide a selected wavelength (e.g., color) or range of wavelengths (e.g., range of colors), for example. It will be appreciated that light rays are similarly reflected from each of the objects to sensors $S_{21}$-$S_{33}$. However, in order to simplify the explanation, these other light rays are not shown.

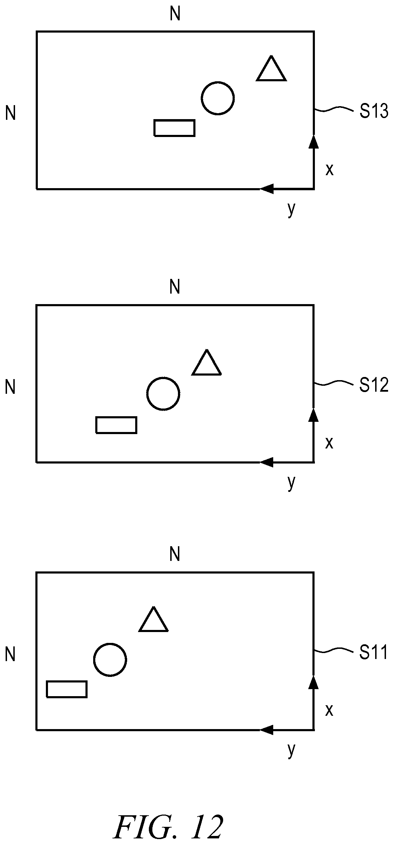

In accordance with modules 401 and 402.1, sensors of the sensor array 210 separately capture images from a world view. Figure 12 is an illustrative drawing representing projections of the three objects of Figure 11 onto the sensors $S_{ij}$ (only $S_{11}$, $S_{12}$, and $S_{13}$ are shown) in accordance with some embodiments. A person of ordinary skill in the art will appreciate that the reflected light rays incident upon the sensors project images of the objects that are in the field of view. More specifically, the rays of light reflected from the objects in the field of view that are incident upon multiple different image sensors of the imager array produce multiple perspective projections of the objects from three dimensions to two dimensions, i.e. a different projection in each sensor that receives the reflected rays. In particular, the relative location of projections of the objects is shifted from left to right when progressing from $S_{11}$ to $S_{12}$ to $S_{13}$. Image sensor pixels that are illuminated by incident light rays produce electrical signals in response to the incident light. Accordingly, for each image sensor, a pattern of electrical signals is produced by its pixels in response to the reflected rays that indicates the shape and location of the image projection within that image sensor.

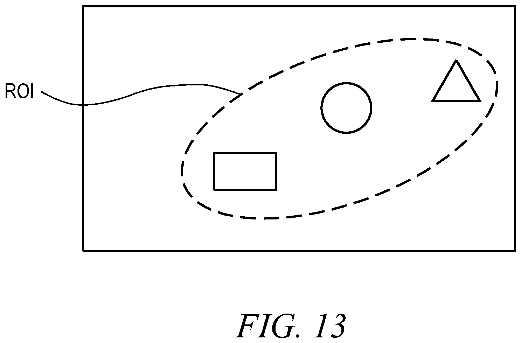

In accordance with module 402.2, a region of interest is selected from the world scene. Figure 13 is an illustrative drawing indicating selection of a region of interest from within the scene. In this example, the triangular-shaped first object, spherical-shaped second object, and rectangular-shaped third object all are in the selected region of interest. This step can be achieved by accepting input from an operator, or it can be automatically performed using a computer configured by software in a prescribed manner, or by combination of operator inputs and automatic software-controlled selection. For example, in some embodiments, the world scene may show an internal cavity of the human anatomy, and the objects may be internal body organs, or surgical instruments, or portions thereof. A surgeon may receive real time visual imagery from within the internal cavity and may see tissue regions of the human anatomy and a portion of the surgical instruments projecting within the body cavity. The surgeon may specify those objects within the field of view for which location information is to be determined through well-known techniques, such as a telestration video marker. Alternatively or in addition to such operator request, an automated process such as an edge detection algorithm can be used to specify a region of interest (ROI).

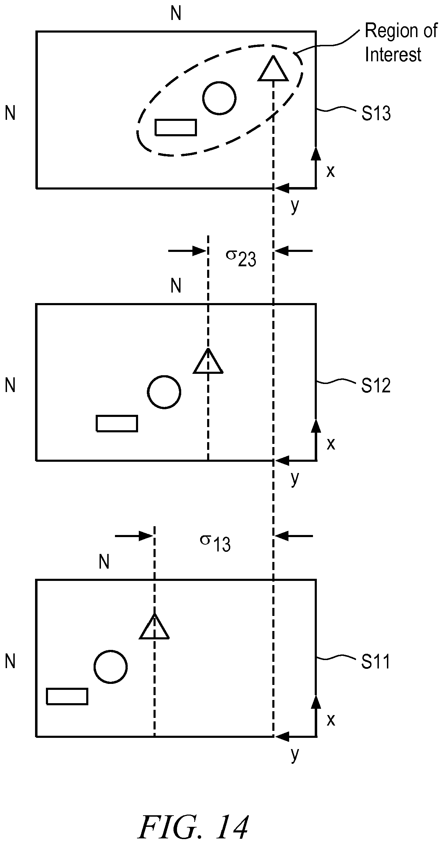

In accordance with module 402.3, a best match is determined between scene images within the region of interest so as to identify pixel locations in different sensors that are illuminated by projections of the same target. Figure 14 is an illustrative drawing showing additional detail about relative geometric offset of the projected images in sensors $S_{11}$, $S_{12}$, and $S_{13}$ in accordance with some embodiments. In accordance with some embodiments, an image from sensor $S_{13}$ is considered to be reference image, and the projections of the objects in the selected ROI are offset to the right by an amount $\sigma_{23}$ pixels in sensor $S_{12}$ relative to their location in sensor $S_{13}$. Similarly, the projections of the objects in the selected ROI are offset to the right by an amount $\sigma_{13}$ pixels in sensor $S_{11}$ relative to their location in sensor $S_{13}$. It will be appreciated that since the FOV viewing axes of sensors $S_{12}$, $S_{11}$ each is offset to the right of the FOV viewing axis of sensor $S_{13}$ (such viewing axes being perpendicular to plane of the sensors), the projected images from ROI are offset to the left in the sensors $S_{13}$ and $S_{11}$ relative to sensor $S_{11}$.

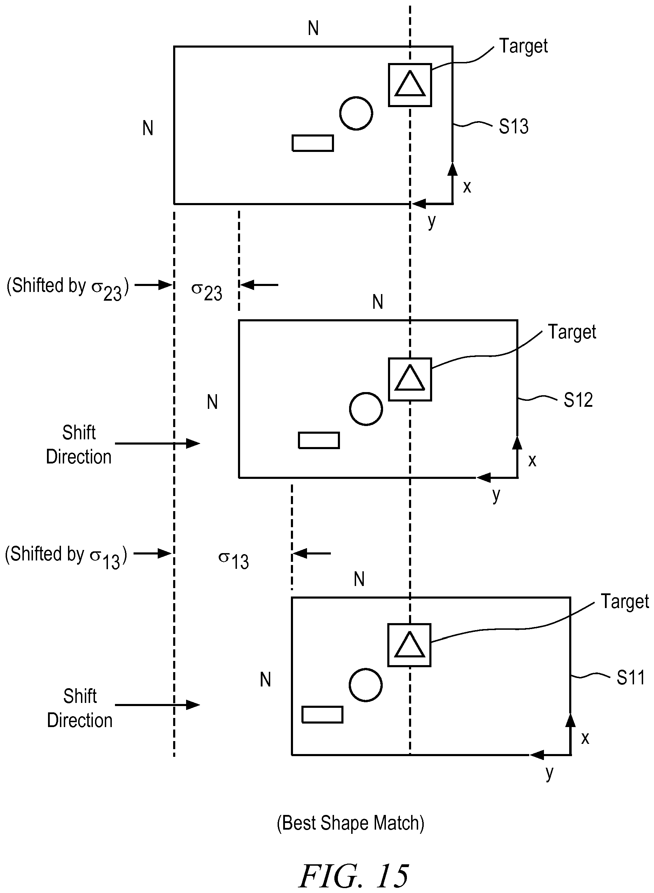

Figure 15 is an illustrative drawing showing the projected images in sensors $S_{11}$ and $S_{12}$ within the ROI shifted to the right to align with the projected images in sensor $S_{13}$ within the ROI in accordance with some embodiments. In the current example, sensor $S_{13}$ is designated to act as a reference sensor. It will be appreciated that other sensors can be chosen for use in determining alignment and geometric dimensions. Projections of the objects within the selected ROI are identified in the designated sensor, e.g., sensor $S_{13}$, and projections in the other sensors, e.g., in sensors $S_{11}$ and $S_{12}$, are shifted until they align with the projection in the designated sensor. In this manner, the corresponding projections of objects within the selected ROI can be identified within the other sensors, together with their offsets relative to the location of the projections in the designated sensor.

In particular, for example, the projections of the three example objects are shifted to the right by an amount $\sigma_{23}$ pixels in sensor $S_{12}$, and the projections of the three example objects are shifted to the right by an amount $\sigma_{13}$ pixels in sensor $S_{13}$. In this illustrative example, in order to simplify the explanation, it is assumed that the projections are offset in the y direction only and not in the x direction, although the same principles apply for x direction projection offsets as between sensors. Moreover, although this example shows a linear offsets, a person of ordinary skill in the art can apply other transformations such as rotation, for example, to align projections that have relative offsets in different sensors.

In accordance with some embodiments for example, two-dimensional (2D) cross-correlation techniques or principal component analysis (PCA), can be used to align the projections within the ROI in $S_{13}$ with the projections within the ROI in $S_{12}$ and to align the projections within the ROI in $S_{13}$ with the projections within the ROI in $S_{11}$. In general, the intent is to best match or align the images from sensors $S_{ij}$ with respect to the image from the sensor designated as reference. More specifically, the projected images within the ROI in $S_{12}$ are shifted and cross-correlated with the projected images within the ROI in $S_{13}$ until a highest correlation coefficient is achieved. Likewise, the projected images within the ROI in $S_{11}$ are shifted and cross-correlated with the projected images within the ROI in $S_{13}$ until a highest correlation coefficient is achieved. Thus, alignment of the projections of the ROI is used to identify the locations of the projections of the ROI in sensors $S_{11}$ and $S_{12}$ by determining the offset between the projection of the ROI in $S_{13}$ and the projection of the ROI in $S_{12}$ and by determining the offset between the projection of the ROI in $S_{13}$ and the projection of the ROI in $S_{11}$.

Example of Candidate Pixel Selection and Refinement

In accordance with module 402.4, candidate pixels are identified within different sensors, which according to the best match process, are illuminated by projections from the same target. Once the projections of objects within the ROI have been identified in each of the sensors $S_{11}$, $S_{12}$, and $S_{13}$, the physical (x, y, z) projections of individual target points within the ROI can be determined relative to the imager array. In accordance with some embodiments, for each of a multiplicity of target points within the ROI, one or more pixels within each of multiple sensors is identified that is illuminated by a projection from the target point. For each such target point, a physical (x, y, z) target point location is determined based at least in part upon the geometric relationships among pixels disposed in different sensors that are determined to be illuminated by projections from the target point.

It will be appreciated that a sequence of target points can be chosen automatically by systematically traversing the ROI (e.g., right to left with a certain step size and up to down with a step size), and a physical (x, y, z) target point location can be determined for each selected target point. Since $S_{11}$ and $S_{12}$ are best matched to $S_{13}$, the traversing is performed inside the shifted regions of interest. Selecting a target involves identifying a pixel in each of sensors $S_{11}$, $S_{12}$, and $S_{13}$ that is illuminated by a projection of the target. Thus, candidate pixels in each of $S_{11}$, $S_{12}$, and $S_{13}$ are identified as being the ones illuminated by a projection of the selected target point.

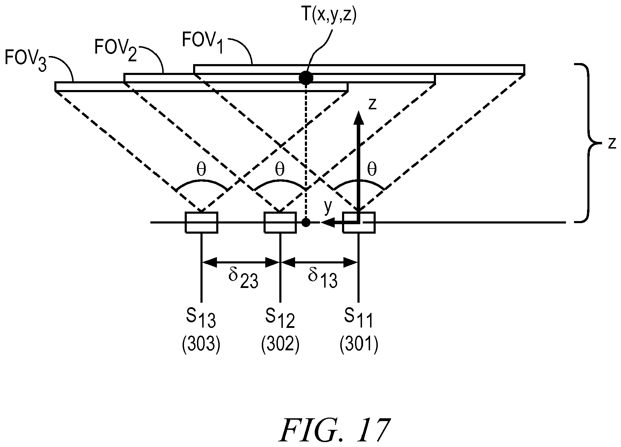

In other words, in order to select a target point T, a pixel is selected in each of the sensors $S_{11}$, $S_{12}$, and $S_{13}$ that is illuminated by a projection of the target point T. It will be appreciated that the (x, y, z) physical location of the target T is unknown at the moment of its selection. Moreover, it will be appreciated that inaccuracy of the above-described alignment process can result in inaccuracy in the determination of which pixels in each sensor are illuminated by the projection of a selected target T. Thus, as explained with reference to Figures 17, 18, and 19, a further determination is made as to the accuracy of the determination as to the pixels in each of $S_{11}$, $S_{12}$, and $S_{13}$ that are illuminated by the projection of a currently selected target T.

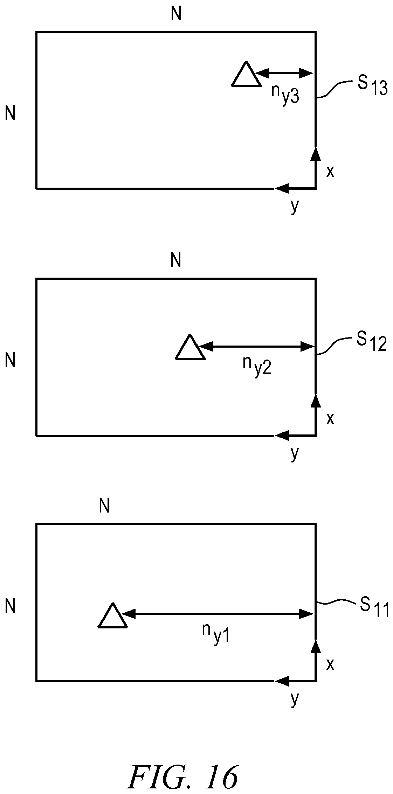

Continuing with the above example, assume that the triangular-shaped first object is the currently selected target point. Figure 16 is an illustrative drawing showing projections of the selected triangle shaped target point onto sensors $S_{11}$, $S_{12}$, and $S_{13}$ in accordance with some embodiments. From these projections, the 2D pixel coordinates for target T are determined, $[(Nx_{11}, Ny_{11}), (Nx_{12}, Ny_{12}), (Nx_{13}, Ny_{13})]$. For simplification, Figure 16 shows only the y-axis pixel coordinates. Using these 2D pixel coordinates, expressions (402.5 - 1) and (402.5 - 2) are applied and $\hat{y}_{12}$ and $\hat{y}_{13}$ computed as part of module 402.5. As part of module 402.6, the norm $|\hat{y}_{12} - \hat{y}_{13}|$ is computed and compared to the acceptable tolerance $\varepsilon$. Similarly, the x-axis pixel coordinates and location estimates are computed and compared against acceptable tolerances. If the condition of module 402.6 is met, then the process proceeds to module 403. Else, it returns to module 402.4 to further refine target candidates.

Referring to Figure 17, there is shown a portion of an imager array that includes sensors $S_{11}$, $S_{12}$, and $S_{13}$ and the selected triangular-shaped first object target point T disposed at location (x, y, z) in physical space. Sensors within an imager array have a known spacing between them, $\delta_{ij}$. The physical position spacing between $S_{11}$ and $S_{12}$ is $\delta_{12}$, and the physical position spacing between $S_{12}$ and $S_{13}$ is $\delta_{23}$. In some embodiments the spacing between all sensors $S_{ij}$ is identical, equal to $\delta$, a constructional specification. Sensors $S_{ij}$ also have a known field of view angle $\theta$.

As explained above, in some embodiments, each sensor is constructed as a 2D imaging element with pixels arranged in a rectangular pattern of rows and columns. Alternatively, pixels can be arranged in a circular pattern, zigzagged pattern, scattered pattern, or an irregular pattern including sub-pixel offsets, for example. The angle and the pixel characteristics of these elements may be identical or, alternatively, may be different from sensor to sensor. However, these characteristics are assumed to be known. In order to simplify the explanation, it is assumed that the sensors are identical, although they may, however, be different.

For simplicity, let us assume that all sensors $S_{ij}$ have N×N pixels. At a distance z from sensor $S_{11}$, the N-pixel width of the sensor expands out to a y-dimension field of view of $S_{11}$ indicated by $FOV_1$. Likewise, at a distance z from sensor $S_{12}$, the y-dimension field of view of sensor $S_{12}$ is indicated by $FOV_2$. Also, at a distance z from sensor $S_{13}$, the y-dimension field of view of sensor $S_{13}$ is indicated by length $FOV_3$. The lengths $FOV_1$, $FOV_2$, and $FOV_3$ overlap each other, signifying that sensors $S_{11}$, $S_{12}$, and $S_{13}$ achieve a 3-way sampling diversity of target T physically located at some (unknown) distance z. Of course, if the sensors are identically built, as assumed in this example, length $FOV_1$, $FOV_2$, and $FOV_3$ will be identical as well. It will be appreciated that the three lengths $FOV_1$, $FOV_2$, and $FOV_3$ all have the same magnitude and are coplanar in that they are at the same (unknown) z-distance from the imager array, although for the purpose of illustration they are portrayed as if they were stacked adjacent to each other.

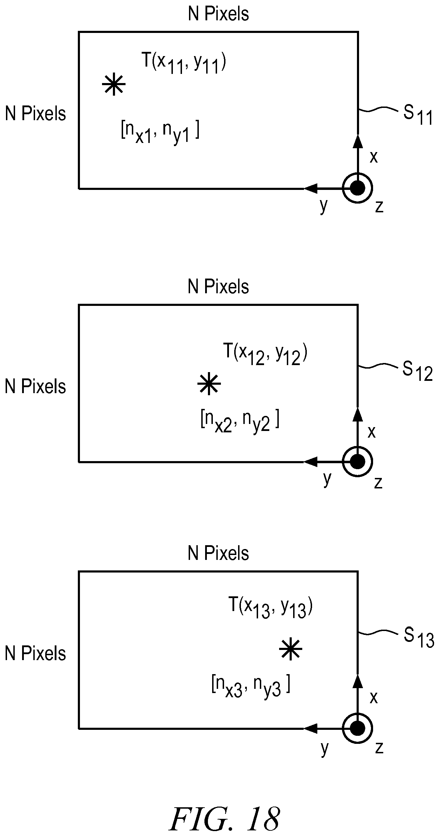

Referring to Figure 18, there is shown an illustrative elevation view of the projection of the currently selected target point T onto the image sensors $S_{11}$, $S_{12}$, and $S_{13}$. For the sake of simplicity, it is assumed that the sensors include geometrically rectangular pixel arrays of size N×N pixels. It is also assumed that the x coordinates of the target T projections are all equal. In other words, it is assumed that for the projections of target T onto $S_{11}$, $S_{12}$, and $S_{13}$, $n_{x1} = n_{x2} = n_{x3}$. To simplify the explanation, it is also assumed that the geometric field of view angle $\theta$ is the same horizontally as it is vertically, $\theta_x = \theta y$. A person of skill in the art would know how to modify the process presented below so that to compute the x, y, and z physical coordinates of target T if any of the above assumptions would change.

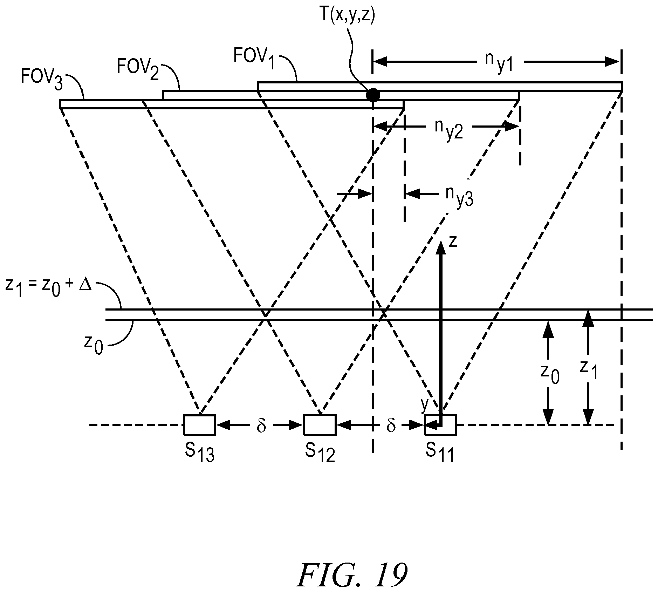

An image of the target T is projected to a physical point within sensor $S_{11}$ at geometric coordinates $(n_{x1}, n_{y1})$, in the plane of the image sensor $S_{11}$. More specifically, the projection of target point T onto sensor $S_{11}$ is located $n_{y1}$ pixels along the y axis, and $_{nx1}$ pixel along the x axis, taken from the origin. An image of the target T is projected to a physical point within sensor $S_{12}$ at geometric coordinates $(n_{x2}, ny2)$ in the plane of the image sensor $S_{12}$. An image of the target T is projected to a physical point within sensor $S_{13}$ at geometric coordinates $(n_{x3}, n_{y3})$ in the plane of the image sensor $S_{13}$. It will be appreciated that pixel locations $(n_{x1}, n_{y1})$ within each sensor are determined relative to origin (0, 0) reference coordinates provided for the sensor. As shown in Figure 17 or Figure 19, a global system of coordinates (x, y, z) is defined and used to reference the target. For example, the origin of such system of coordinates may be placed, without limitations, at the geometrical center of sensor $S_{11}$.

Referring to both Figure 16 and Figure 18, it can be seen that the y pixel distance of the projection of the target is different in each sensor. The projection of a currently selected target T is disposed $n_{y1}$ pixels to the left of the origin in $S_{11}$. The projection of the selected target T is disposed $ny2$ pixels to the left of the origin in $S_{12}$. The projection of the selected target T is disposed $n_{y3}$ pixels to the left of the origin in $S_{13}$. As mentioned above, to simplify the explanation, it is assumed that the projection of the target falls at the same x pixel distance from the origin in all three sensors.

Referring to Figure 19, there is shown the disposition of the currently selected target T relative to sensors $S_{11}$,

$S_{12}$ and $S_{13}$ as described above with reference to Figure 17 and also showing y-direction pixel offsets for the candidate pixel in each of the sensors. It will be understood that the drawings of Figure 19 present physical structures and an analytical framework for determining the (x, y, z) physical coordinates of the selected target point T. At an (unknown) distance z from the imager array plane, the y-direction field of view for each sensor extends over a length marked as $FOV_1$. This length, $FOV_1$, corresponds to the maximum pixel width of the sensor, which is N pixels, in some embodiments. Given that the working assumption was that the sensor has a field of view that is symmetric in the x and y directions, the length would also be $FOV_i$ vertically, along the x axis.

Recall that the candidate pixel selections are made based at least in part upon a correlation process that can have a level of uncertainty than can result in inaccuracy in determination of the physical location of the selected target. Thus, a further check of the accuracy of the target projection candidate selections, in accordance with some embodiments, is made as follows.

Example of Determining Target's Physical (x, y) Location and Checking Accuracy of Target Projection Candidate Selection

In accordance with module 402.5, two or more two-dimensional ($N_x$, $N_y$) coordinate values are computed for the selected target to determine whether the candidate pixels actually are illuminated by a projection from the same target. Based on the assumptions discussed above and placing the origin of the 3D system of coordinates at the center of sensor $S_{11}$, the imager array and currently selected target T in the example in Figure 19 have the following relationships:

$$z = \frac{N \cdot \delta}{2 \cdot (n_{y1} - n_{y2}) \cdot \tan(\frac{\theta}{2})} \qquad (1)$$

$$y = \frac{2n_{y1} - N}{2(n_{y1} - n_{y2})} \cdot \delta \qquad (2)$$

$$x = (\frac{2n_{x1}}{N} - 1) \cdot z \cdot \tan(\frac{\theta}{2}) \qquad (3)$$

Where:

N is the pixel dimension of the imaging sensors;

$n_{x1}$ is the position of a target point T expressed in number of pixels from the origin of the $S_{11}$ plane in the x direction;

$n_{y1}$ is the position of the target point T expressed in number of pixels from the origin of the $S_{11}$ plane in the y direction;

ny2 is the position of the target point T expressed in number of pixels from the origin of the $S_{12}$ plane in the y direction; and

ny2 is the position of the target point T expressed in number of pixels from the origin of the $S_{12}$ plane in the y direction;

0 is the angle of the field of view.

Moreover, if performing the same math using sensors $S_{11}$ and $S_{13}$ and given that the separation between $S_{11}$ and $S_{13}$ is 2δ, we obtain:

$$z = \frac{2 \cdot N \cdot \delta}{2 \cdot (n_{y1} - n_{y3}) \cdot \tan(\frac{\theta}{2})} \qquad (4)$$

$$y = \frac{2n_{y1} - N}{2(n_{y1} - n_{y3})} \cdot 2\delta \qquad (5)$$

$$x = (\frac{2n_{x3}}{N} - 1) \cdot z \cdot \tan(\frac{\theta}{2}) + 2\delta \qquad (6)$$

Where:

$n_{x3}$ is the position of a target point T expressed in number of pixels from the origin of the $S_{13}$ plane in the x direction; and

$n_{y3}$ is the position of the target point T expressed in number of pixels from the origin of the $S_{13}$ plane in the y direction.

Thus, determination of the physical x coordinate of the selected target T can be determined based upon expressions (3) or (6). A determination of the physical y coordinate of the selected target T can be determined based upon expressions (2) or (5). A determination of the physical z coordinate of the selected target T can be determined based upon equations (1) or (4).

More generally, in accordance with module 402.6, a determination is made as to whether the computed 2D coordinate values indicate that the candidate pixels are illuminated by a projection from the same target. It will be appreciated that a more reliable determination of the physical (x, y, z) coordinates of the target T can be obtained through the use of two formulations for each coordinate. For example, the y coordinate for the target T can be determined using both formulations (2) and (5). If the resulting y coordinate values computed using the two formulations differ by more than some acceptable tolerance value, $\varepsilon_y$, then a determination can be made that the matching process failed to resolve the offset between projections in the different sensors with sufficient accuracy, and as result that the candidate pixels do not correspond in that they do not receive projections from the same target T. In the event of a failure of the y computations to match, another iteration of the matching process may be performed in an effort to make an improved selection of candidate pixels within the sensors that each corresponds to a selected target T. It will be appreciated that the computed y values are unlikely to be equal since the different perspective projections onto different sensors can differ due to parallax effects, for example. Therefore, an acceptable tolerance value is prescribed according to the intended application. For surgical imaging applications, an $\varepsilon$ of 0.1 - 0.3 mm typically offers an acceptable Q3D accuracy. A person of skill in the art may define different acceptable tolerance levels .

Given the assumed sensor symmetry around the x and y axes, persons skilled in the art will appreciate that the same kind of determination can be made for the x coordinates of the target T using formulations similar to those in (2) and (5), but using $n_{xi}$ instead of nyi. Formulations (3) and (6) cannot be used part of 402.5 and 402.6 because they require knowledge of the z coordinate. However, the essence of modules 402.5 and 402.6 is to determine the correct target projections on the planes of sensors $S_{11}$, $S_{12}$ and $S_{13}$. For this purpose formulations (2) and (5), adjusted for x and y axes, are sufficient. The complete set of coordinates (x, y, z) is computed part of modules 403 and 404, as described below.

Example of Determining Target's Physical z Location

As illustrated in Figure 19, in accordance with modules 403 and 404, an initial estimate for the z coordinate, $z_0$, is used to initiate the computation process. This initial value is defined automatically, according to the medical application. The medical application defines the intended world view to be visualized. The initial value $z_0$ starts at the edge of the field of view closest to the endoscope. Referring to Figure 8, for a Q3D application involving surgical endoscopy, $z_0$ can be 1 - 5 mm off the distal end 208 of the Q3D endoscope 202, for example. Such initial estimate generally is sufficient for this application as it is unlikely to have any tissues or surgical instruments reside in such close proximity to the Q3D endoscope. Next, value $z_0$ is plugged into formulations (3) and (6). Given that the x coordinate of the target is unique, if $z_0$ were the true and correct z coordinate of the target then formulations (3) and (6) would yield identical values, or approximately equal, within an acceptable level of tolerance, $\varepsilon_x$.

$$|x_{(3)} - x_{(6)}| < \varepsilon_x \qquad (7)$$

If (3) and (6) are outside an acceptable tolerance $\varepsilon_x$, then the iteration continues and a new estimate for z is tried, $z_1$. In accordance with some embodiments, the new estimate is defined automatically. For example, $z_1 = z_0 + \Delta$ , where $\Delta$ is the size of the iteration step. In general, at $k^{th}$ iteration $z_k = z_{k-1} + \Delta$ . The iterative process stops when condition (7) is met. A smaller $\Delta$ yields increased accuracy in determining the correct target coordinates, but it would also require more computational time to complete the process, hence an increased latency. An increased latency may result in delays between surgical instrument movement and its visualization by the operating surgeon. In other words, the surgeon may perceive the system as lagging behind commands. For a surgical viewing space of 20 - 30 cm of depth, a $\Delta$ of 0.1 - 0.3 mm may be sufficient. Of course, a person skilled in the art would know to balance the size of $\Delta$ against the computational required to complete the iterative process.

The above explanation has been simplified for presentation reasons and, therefore, it included only three sensors, $S_{11}$, $S_{12}$, and $S_{13}$. In general, more sensors can be used to increase the accuracy of Q3D coordinate computations but also to reduce the overall number of iterations. For example, if more than three sensors are used, preferably an array of $3 \times 3$ sensors, then methods such as the steepest gradient may be employed to trend the direction of estimation errors made by modules 402.5 and 403. The iterative step size and direction can then be adjusted to match the progression towards the local extreme of the 3D error gradient surface.

Guiding Endoscopic Surgery with Q3D Information

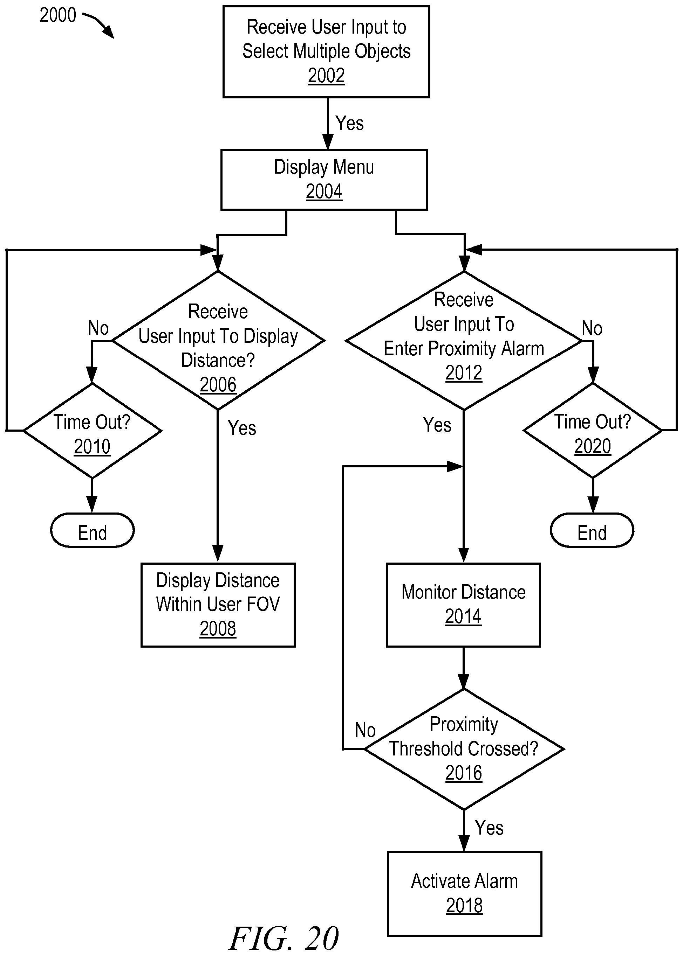

Figure 20 is an illustrative flow diagram representing a first process 2000 to use Q3D information during a surgical procedure in accordance with some embodiments. Computer program code configures the computer 151 to perform the process 2000. Module 2002 configures the computer to receive user input to select at least two objects within a surgeon's field of view when looking into the viewer 312. Module 2004 configures the computer to display a menu on a computer console in response to receipt of a user selection. Decision module 2006 configures the computer to determine whether user input to the menu is received to display a distance. In response to a determination that user input is received to display a distance, module 2008 configures the computer to display a numerical distance within the video image in the surgeon's field of view. Decision module 2010 configures the computer to wait for a prescribed time interval for receipt of user input to select distance display and to end operation of decision module 2006 in response to no receipt of user input within a "time out" interval.

Decision module 2012 configures the computer to determine whether user input to the menu is received to enter a proximity alarm limit. In response to a determination that user input is received to enter a proximity threshold, module 2014 configures the computer to use Q3D information to monitor proximity between two or more objects within the surgeon's field of view. Decision module 2016 determines whether the proximity threshold has been crossed. In response to a determination that the proximity threshold has been crossed, module 2018 configures the computer to activate an alarm. The alarm may include a sound, a visual queue such as a blinking light, locking of instrument movement to avoid collision, or other haptic feedback. In response to a determination that the proximity threshold has not been crossed, control flows back to monitoring module 2014. Decision module 2020 configures the computer to wait for the prescribed time interval for receipt of user input to enter the proximity threshold and to end operation of decision module 2012 in response to no receipt of user input within the "time out" interval.

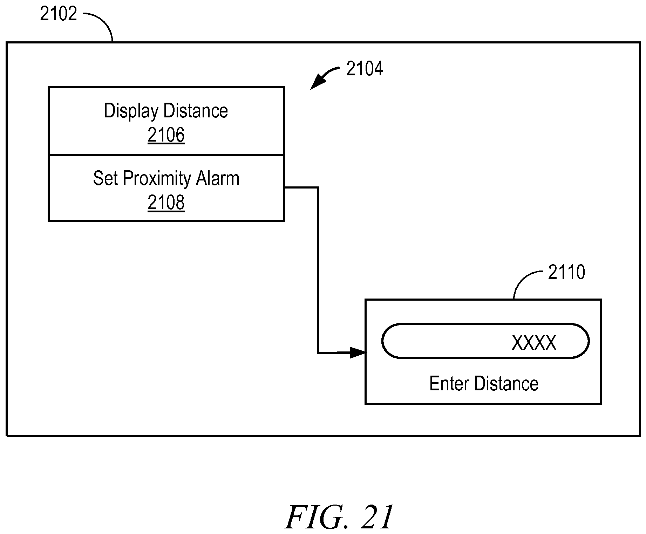

Figure 21 is an illustrative drawing showing menu selections displayed on a display screen 2102 in accordance with the process of Figure 20 in accordance with some embodiments. The display screen 2102 includes a viewing monitor associated with the computer 151. Alternatively, the display screen 2102 may include a region of the viewing elements 401R, 401L of the viewer 312. In response to user input, module 2004 causes the display of a menu 2104 that includes a first menu item "Display Distance" 2106 and a second menu item "Set Proximity Alarm" 2108. In response to user input to select the "Display Distance" menu item 2106, module 2008 causes a display of Q3D distance between two or more objects. Referring again to Figure 4, there is shown a display of a Q3D distance "d_Instr_Trgt" between an instrument 400 and target displayed using module 2008. In response to user input to select the "Set Proximity Alarm" menu item 2108, an "Enter Distance" UI input 2110 is displayed that includes a field in which a user can enter a proximity distance threshold value, e.g., one cm. In an alternative embodiment (not shown), a default proximity threshold may be set in advance for all instruments, and a user may change the proximity threshold using the menu of Figure 21, for example. In the alternative embodiment, a user can choose to elect the default threshold value rather than enter a threshold value. In some embodiments, a user can select both to display the distance and set a proximity alert.

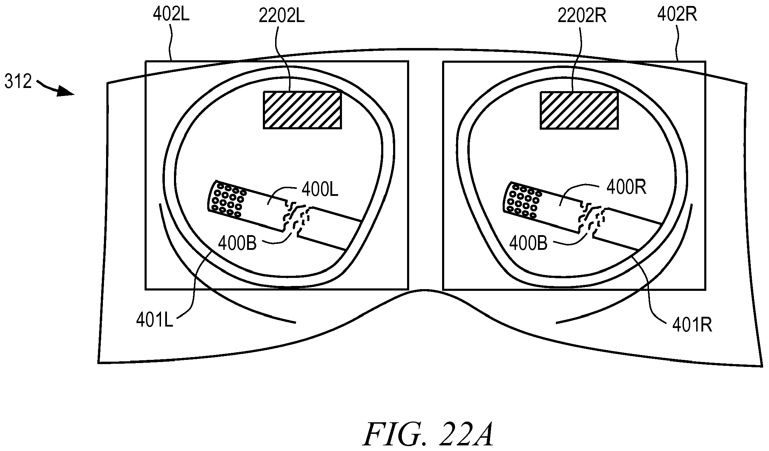

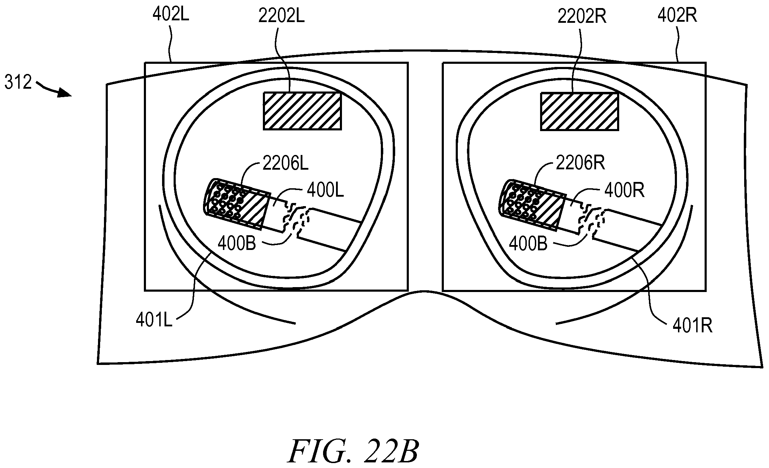

Figures 22A-22B are illustrative drawings representing certain details of receiving user input in accordance with the process of Figure 20 in accordance with some embodiments. Figure 22A shows example first highlighting areas 2202L, 2202R of a target 410L, 410R, such as body tissue, which can be created using video marker tool, such as telestration, or using the surgeon console manipulating control input devices 160 of Figure 4. Figure 22B shows example second highlighting areas 2206L, 2206R of an instrument tip 400L, 400R, which can be created using the video marker tool. In operation in accordance with some embodiments, a user creates the first highlighting areas 2202L, 2202R. Next, the user creates second highlighting areas 2206L, 2206R of the instrument tip 400L, 400R using video marker tool. It will be understood that the order in which items are highlighted is unimportant. The user then actuates a selector (not shown) (e.g., press the ENTER key) to enter the selection. Module 2002 interprets the received user input as selection of the target image 410L, 410R and the instrument image 400L, 400R.

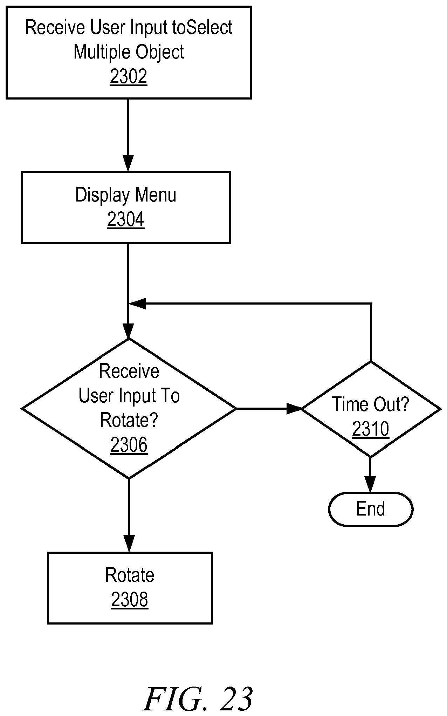

Figure 23 is an illustrative flow diagram representing a second process 2300 to use Q3D information during a surgical procedure in accordance with some embodiments. Computer program code configures the computer 151 to perform the process 2300. Module 2302 configures the computer to receive user input to select an object within a surgeon's field of view when looking in to the viewer 312. For example, referring again to Figure 22A, user input is shown received to create the second highlighting areas 2206L, 2206R of the instrument tip 400L, 400R using the video marker tool. User input (not shown) is received to actuate a selector (not shown) (e.g., press the ENTER key) to enter the selection of the image of the instrument tip 400L, 400R.

Returning once again to Figure 23, in response to receipt of a user selection, module 2304 configures the computer to display a menu on a computer console. Decision module 2306 configures the computer to determine whether user input to the menu is received to rotate an image of a selected object. In response to a determination that user input is received to rotate an image, module 2308 configures the computer to display rotate the image to show a different three-dimensional perspective of the object. Decision module 2310 configures the computer to wait for a prescribed time interval for receipt of user input to rotate an image and to end operation of decision module 2306 in response to no receipt of user input within a "time out" interval.

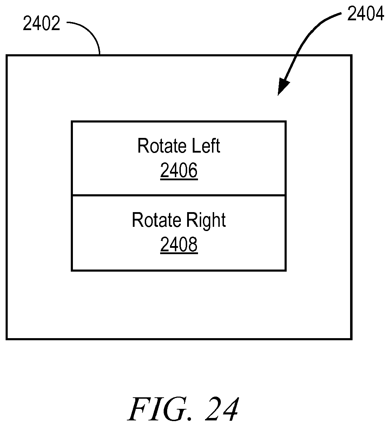

Figure 24 is an illustrative drawing showing menu selections displayed on a display screen 2402 in accordance with the process of Figure 23 in accordance with some embodiments. The display screen 2402 includes a viewing monitor associated with the computer 151. Alternatively, the display screen 2402 may include a region of the viewing elements 401R, 401L of the viewer 312. In response to received user input, module 2304 causes the display of a menu 2404 that includes a third menu item "Rotate Left" 2406 and a fourth menu item "Rotate Right" 2408. In response to user input to select one or the other of the third or fourth menu items 2406, 2408, module 2308 uses the causes a rotation of the 3D model created and stored pursuant to module 407 of Figure 9. It will be appreciated that the amount of rotation may be limited to a few degrees, less than 30 degrees for example, since the sensor imager array 210 has a limited overall field of view.

Q3D Model Alignment with 3D Image representation

Figure 25 is an illustrative drawing showing details of a process to produce a 3D rendering of the Q3D model in accordance with some embodiments. Once the transformed Q3D model has be computed, it can be displayed on the 3D display 110 of the system. A number of 3D video algorithms and hardware implementations can be used for this purpose. The computer module 2502 configures the system of Figure 8, which comprises the video processor 104, controller 106, and display driver 109, to separate the Q3D model into a right-eye view 2504R and left-eye view 2504L, given an average human interpupillary distance (IPD) of 62-65 mm. To achieve this step, known details of stereoscopy and human IPD are applied. In accordance with some embodiments, module 2506 configures the 3D video controller 106 of 3D display driver 109 to alternately switch between providing video frames to the left-eye 2508L and providing frames to the right-eye 2508R at known frame rates in order to give the viewer the impression of a 3D scene. The approach described in Figure 25 employs 3D viewing goggles, such as those present on a teleoperation surgery system such as that described in Figures 4-6. However, other 3D video display mechanisms can be used. Patents U.S. Patent No. 4,562,463 issued to Lipton and U.S. Patent No. 6,008,839 issued to Nagele et al. provide additional implementation details.

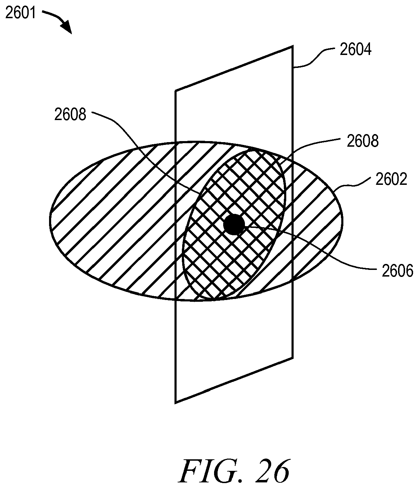

Figure 26 is an illustrative perspective view drawing representing an example of a three-dimensional (3D) volumetric image representation 2601 of an example anatomical structure 2602. The image includes image information representing structures throughout the 3D volume of the anatomical structure 2602. For example, without limitation, the external 3D image can be an MRI, CT, PET, or ultrasound image. The 3D image representation provides a 3D representation of the anatomical structure 2602. In order to simplify the following discussion, hereafter we will refer to MRI/CT images. However, the techniques described are applicable to other 3D image types, such as PET or ultrasound, for example. The 3D image representation 2601 can be viewed in two-dimensional (2D) cross-sectional slices 2604 (only one shown). More specifically, a cross-sectional slice portion of the 3D image can be selected for display on a computer display screen to show cross-sectional representations of the anatomical structure at different angular cross-sections and at different depths from the surface of the structure. The illustrative example anatomical structure 2602 includes a surface structure at its surface (surface contour 2608 shown) and includes a sub-surface structure 2606 that may be a target structure of interest in a surgical procedure, for example.

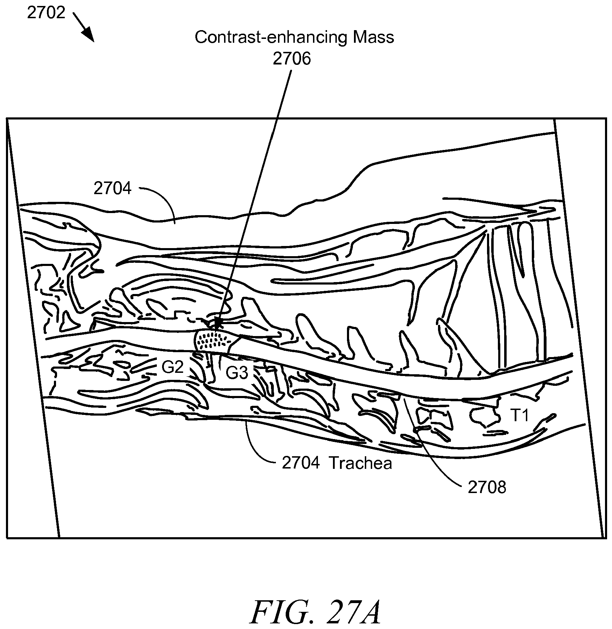

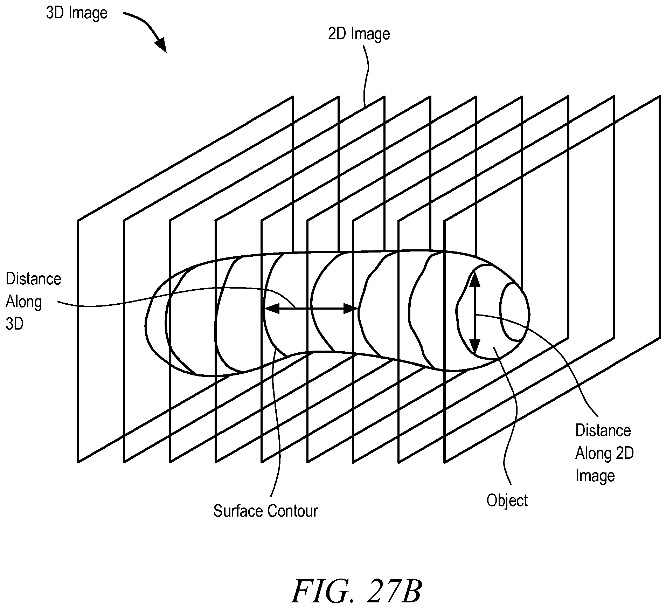

Figure 27A is an illustrative drawing showing a 2D cross section of a 3D MRI/CT representation that shows surface structures 2704 of an anatomical structure 2702 and sub-surface structures 2706 of the anatomical structure. More specifically, the anatomical structure 2702 includes a portion of a patient's spine 2708, which is a subsurface structure, and a tumor mass 2706, which is another subsurface structure localized on the patient's spine 2708 . As shown in Figure 27, dimensional information can be readily determined, such as the size of structures represented within the 3D representation and distances between anatomical objects represented within the 3D representation. The 3D MRI/CT representation is stored in a computer readable storage device.

Typically, in practice for example, a medical specialist such as a radiologist manually identifies a candidate target structure by viewing multiple 2D image slices from a 3D MRI/CT representation. Sometimes, for example, an identified target is confirmed with another imaging modality. For example, ultrasound imaging is frequently used as a confirmatory modality in conjunction with MRI or CT images. These 3D visual imaging techniques provide dimensional/quantitative information about the location of a target structure internal to an anatomical structure and its relationship to surface structures of the anatomical structure. For example, Figure 27A shows not only the depth of the tumor mass, which is the target structure internal to the anatomical structure in this example, but also shows the contour of the surface of the anatomical structure immediately above the tumor. As explained more fully below, during a surgical procedure, such surface contour information provided by a 3D MRI/CT representation can be correlated with Q3D information so as to more accurately pinpoint alignment between visible surface contours of the anatomical structure and the target structure that is internal to the anatomical structure, and therefore, not visible.

Figure 27B is an illustrative drawing representing a 3D view of an anatomical object and associated 2D image slices through the object created using CT or MRI techniques, for example. The anatomical object includes an inner subsurface object, which may be a tumor for example. The anatomical object has an external surface contour. One or more of the 2D image slices contain image slices of the internal subsurface object. The 2D image slices can be aligned with the a Q3D image of the object using fiducials, such as structures visible in both the 2D slices and a view of the external surface contour of the object. The subsurface object can be made visible in a ghost image overlaying an image of the physical object so as to inform surgeon of location, distance and dimension information during exploration or surgery, for example.

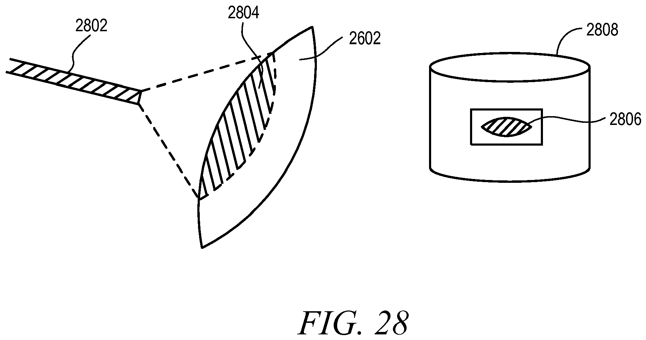

Figure 28 is an illustrative drawing showing a Q3D endoscope 101C positioned to capture image information corresponding to an external surface portion 2804 of the anatomical structure 2602 of Figure 26 in accordance with some embodiments. The captured Q3D information associated with surface portion 2804 represents a 3D surface contour of portion of the anatomical structure 2602 within a field of view (FOVe) of the Q3D endoscope. A Q3D model 2806 representing the captured Q3D surface contour information 2804 is stored in a computer-readable storage device 2808.

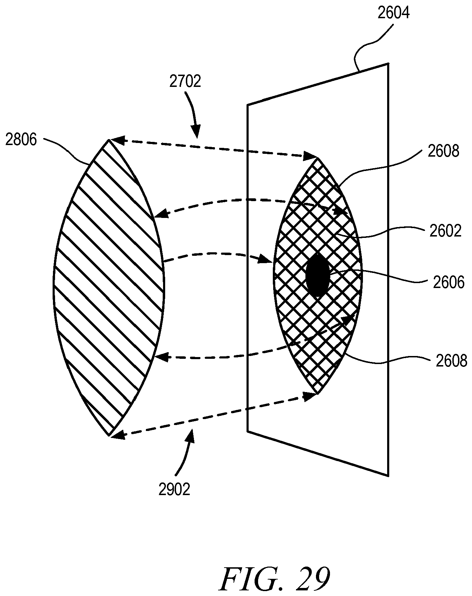

Figure 29 is an illustrative drawing conceptually representing a process of aligning the example 3D MRI/CT volumetric image representation 2601 of the anatomical structure 2602 of Figure 26 with the example Q3D model 2806 of Figure 28 in accordance with some embodiments. The Q3D model 2806, which comprises the surface contour information for the anatomical structure 2602, is aligned with corresponding surface contour information portions of the 3D MRI/CT representation 2601. For example, elements of cross section 2604, such as the surface contour 2608, are aligned with corresponding elements from Q3D model 2806. In accordance with some embodiments, the Q3D model 2806 represents surface contour of an anatomical structure 2602 in terms of distance of points on the external surface of the anatomical structure from the endoscope 101C. Whereas, the outer surface of the anatomical structure 2602 is represented in the 3D MRI/CT in terms of outer edge portions 2608 of 2D image slices of the anatomical structure. Alternatively, 3D surface data, such as 2608, can be obtained by extracting it from the 3D MRI/CT volumetric model 2601. If input means 112 provide the visual representation of structure 2602 as a set of 2D images, such as cross section 2604, processor 106 may be programmed to combine these images so as to blend a number of component images into a single image and to create the 3D volumetric representation 2601. In other embodiments, input means 112 receive the 3D volumetric representation 2601 from the external imaging system (MRI, CT, PET, ultrasound, etc.). The alignment of the Q3D model with the MRI/CT 3D representation involves determining a common group of external fiducial points (not shown) represented in the two image representations, and then aligning the two image representations according to the common external fiducial points. A Q3D-MRI/CT combination representation can be produced based upon the alignment of the Q3D model with the MRI/CT image representation.

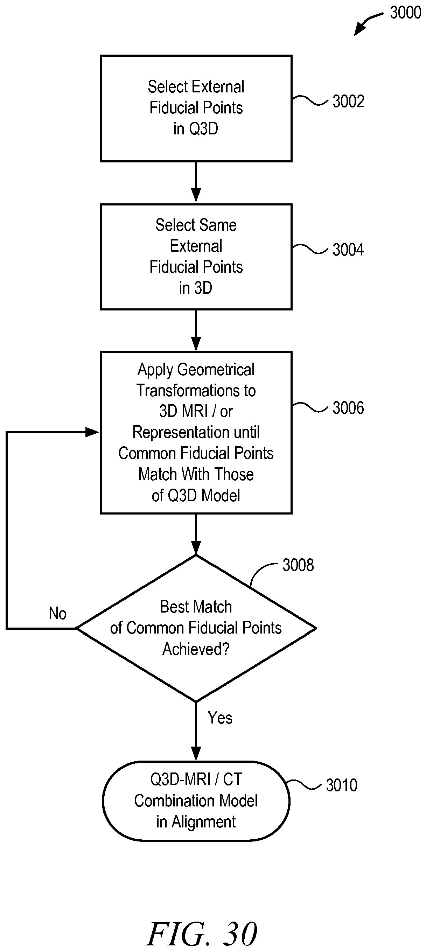

Figure 30 is an illustrative flow diagram showing details of a process 3000 to align a Q3D model with a 3D MRI/CT representation in accordance with some embodiments. Module 3002 configures a processor, such as 106 of Figure 8, to select fiducial points in a Q3D model. Module 3004 configures the processor to select the same fiducial points in a 3D MRI/CT image representation. Module 3006 applies a geometric transformation of the orientation of the 3D MRI/CT image representation until its selected fiducial points align with those selected for the Q3D model. Different geometric transformations are determined using module 3006 until decision module 3008 determines that a best match of the selected fiducial points has been achieved. Module 3010 provides as an output an indication of the best alignment of the Q3D model and the 3D MRI/CT image representation.

Referring again to Figure 27A, for example, an endoscope may be positioned adjacent to the surface of the anatomical structure so as to be positioned to capture Q3D information used to produce a Q3D model of the visible surface contour of the vertebra surrounding the internal target structure. The surface contour of those same vertebra are represented in the 2D slice of an MRI/CT representation shown in Figure 27A. The fiducial points may be selected as contours of the common vertebra visible in the two image sets, i.e., in the Q3D model and in the 3D MRI/CT representation. The geometrical transformation, for example, may include rotations and translations of the 3D MRI/CT image until the vertebra contours represented in the 3D MRI/CT image achieve a best match alignment with vertebra contours represented in the Q3D model, at which point the two image sets are determined to be aligned. For example, determining a best match may involve cross-correlating 2D contours of the 3D MRI/CT image with contours of the Q3D model until a maximum cross-correlation factor is achieved. Alternatively, in accordance with some embodiments, a best match can be determined by iterating transformation of the 3D MRI/CT image until a sum of the Euclidean distances between a pair of points on the selected vertebra, represented in the Q3D model and in the 3D MRI/CT representation, is minimized or decreases below an acceptance threshold.

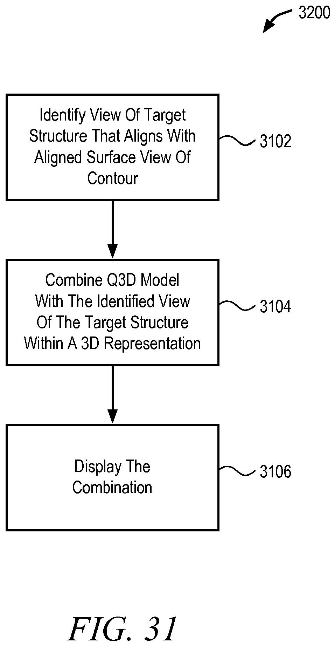

Figure 31 is an illustrative flow diagram representing a process 3100 to generate a visual display based upon a Q3D-MRI/CT combination model in accordance with some embodiments. Module 3102 configures a computer system, or processor such 106 of Figure 8, to identify a view of the target structure within the 3D MRI/CT representation that aligns with the aligned surface of the 3D MRI/CT representation. It will be appreciated that the process 3100 results in alignment of the Q3D model with a surface portion of the 3D MRI/CT representation from the perspective of the Q3D endoscope. Thus, it will be appreciated that in accordance with some embodiments, module 3102 identifies a view of the sub-surface structure that also aligns with this same Q3D endoscope perspective. Module 3104 configures a computer system to combine a visual view of the Q3D model with the identified view of the target structure within the 3D MRI/CT representation. Module 3106 configures the display system of Figure 25 to display a visual view showing the combined views. Alternatively, the output of module 3104 may be stored by module 407 of Figure 9 and provided for further manipulation to external devices or to other processors of system 152. For example, the combined Q3D-MRI/CT model may be used to guide control of surgical instruments 101 of Figure 6 so to achieve a desired clinical outcome. It will be appreciated that the combined view portrays both the surface contour of the anatomical structure within the FOVe of the Q3D endoscope and the sub-surface target structure. Moreover, the alignment between sub-surface target structure and the externally visible surface contour is portrayed accurately in the combined view.

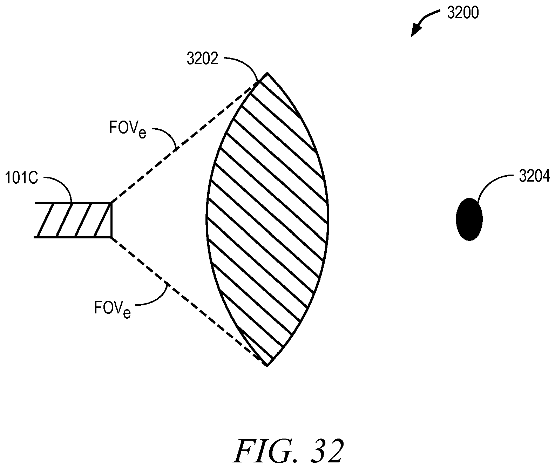

Figure 32 is an illustrative drawing representing a Q3D-MRI/CT combination model 3200 in accordance with some embodiments. The Q3D-MRI/CT combination model 3200 includes Q3D information 3202 from the Q3D model 2806 that is indicative of the surface portion 2804 of the anatomical structure 2602 that is currently visible from the perspective of the Q3D endoscope 101C and also includes image information 3204 for the internal sub-surface target structure 2606 that is not currently visible by the Q3D endoscope. Significantly, the relationship between the surface contour portion 3202 and sub-surface structure image information 3204 within the Q3D-MRI/CT combination model 3200 provides an accurate representation of the alignment of the surface portion 2608 portion and the internal sub-surface target structure 2606. In order to illustrate this representation of the alignment, a positional relationship between the surface contour portion 3202 and the endoscope 101C and its field of view (FOVe) are shown in Figure 32. By combining the external 3D image (e.g., MRI, CT, PET, ultrasound) 2601 with the Q3D model 2806, a Q3D-MRI/CT combination model 3200 is constructed. Referring to Figures 29 and 32, in the Q3D-MRI/CT combination model 3200, the image information 3204 representing the internal sub-surface structure 2606 is aligned with image information 3202 corresponding to visible anatomic details 2804 visible by the Q3D endoscope 101C, real-time during the procedure. Thus, the combined representation includes a merging of the surface contour information of the Q3D model with the selected sub-surface structure representation selected according to the process of Figure 30. More specifically, the combined Q3D-MRI/CT model 3200 comprises a collection of (x, y, z) coordinates in reference to details visible from a viewpoint of the endoscope 101C.

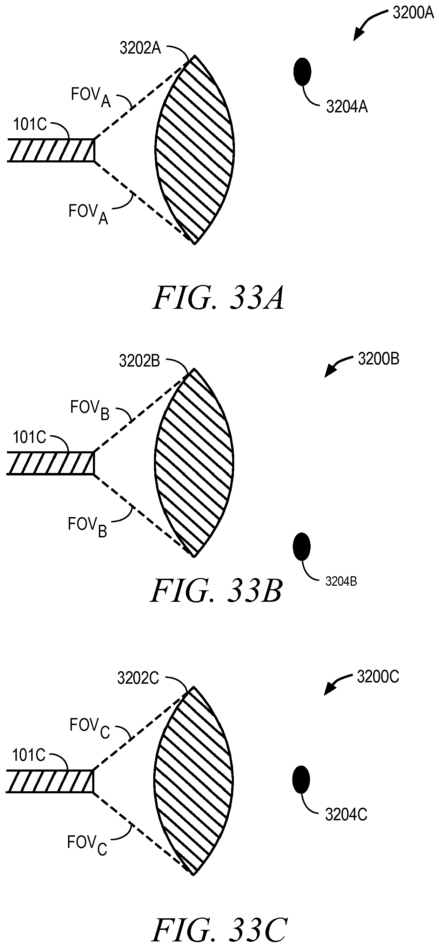

Figures 33A-33C are illustrative drawings representing three different Q3D-MRI/CT combination models 3200A, 3200B, and 3200C based upon three different surface regions of the anatomical structure being within the field of view of the endoscope 101C in accordance with some embodiments. It will be understood that the position of the endoscope 101C can be changed to change its field of view FOVe. In accordance with some embodiments, a user can observe the location of a target structure within a current field of view, and based upon its observed position, the user can alter the position of the endoscope to change the position of the target to a desired location within the FOVe. For example, assume that a user desires the target to be centered in the FOVe as shown in Figure 33B. In that case, the user observing the target position 3204A in Figure 33A can move the endoscope 101C downward to arrive at the target position 3204B in Figure 33B. Alternatively, the user observing the target position 3204C in Figure 33C can move the endoscope 101C upward to arrive at the target position 3204B in Figure 33B. Once the desired orientation of endoscope 101C is achieved, the surgeon can instruct system 152 to move other instruments 101 according to clinical goals related to sub-surface target 3204. For example, if sub-surface target 3204 were a tumor, applicable instruments 101 are optimally aligned and manipulated to allow the surgeon to reach to the tumor and to operate on it, in spite of the fact that the tumor is not directly visible.

In accordance with some embodiments, a user can visualize the combined Q3D-MRI/CT model using the 3D viewer 110, as described in detail in Figure 25. In other words, a visual 3D view of the combined Q3D-MRI/CT model is displayed in which the surface contour from the perspective of the Q3D endoscope is visible and the sub-surface target structure represented within the 3D MRI/CT image also is visible. It will be appreciated that a user, therefore, can discern a physical relationship between the visible surface contours that are within the view perspective of the Q3D endoscope and the target sub-surface structure. As discussed above, portraying this relationship to a user can aid the user in performing a surgical procedure such as by informing the user, such as a surgeon, of the best location to make an incision to access the target structure, for example. Portraying the relationship also can inform a user of adjustment in the position of an endoscope or other surgical instrument required to better observe or access the target structure, for example.

In another aspect, continuous Q3D information about a surface structure, about a sub-surface structure, or about both a surface structure and a subsurface structure can be used to continually update an earlier-obtained volumetric model (e.g., a volumetric MRI/CT image rendering) that includes the surface structure, the sub-surface structure, or both the surface structure and the sub-surface structure. Therefore, as the surgical environment changes (e.g., due to tissue position shifting, tissue retraction, and the like), a continuously updated model is available from the combined pre-operative image information and the most recent current Q3D image by continuously applying the process above. As a result, the surgeon has access to the continuously updated model to more precisely navigate to tissue of interest identified in the preoperative image.

In still another aspect, the capability to continuously update a preoperative volumetric image model with current 3D information as described above allows a surgeon to more accurately navigate along a complex tissue pathway. Such a pathway may be via one or more open body lumens, or it may be defined along one or more tissue planes. The pathway is first identified in a 3D rendering from pre-operative images, and then during surgery the identified pathway is continuously updated by using Q3D information as described above.

The above techniques for alignment of a Q3D model with a 3D MRI/CT model also apply in general to alignment of a Q3D model with a fluorescent light image that portrays a combination of surface contours and sub-surface structures. For example, fluorescent imaging is used part of the *FireFly*® product line supplied with the *da Vinci* Si® or Xi® surgical systems commercialized by Intuitive Surgical, Inc. A fluorescent die may be introduced to an anatomical structure. For example, Indocyanine green (ICG) is a fluorescent dye which is used in medicine as an indicator substance (e.g., for photometric hepatic function diagnostics and fluorescence angiography) in cardiac, circulatory, hepatic and ophthalmic conditions. The fluorescence spectrum of ICG is between 750 nm and 950 nm. ICG dye is injected into the blood stream of the patient and it binds to albumin protein. Certain sub-surface targets, such as tumors, have an increased blood supply. Therefore, such sub-surface targets have an increased temporary supply of ICG, until the dye is eliminated by the body. An excitatory laser light (e.g., 803 nm) may be integrated with endoscope 101C of Figures 6 or 7 and used to excite the dye. The dye will then emit fluorescent signal within its excitation spectrum (e.g., 830 nm). The fluorescence signal is received by the Q3D endoscope 101C, processed by the CCU 104 and processor/controller 106, both of Figure 8. A Q3D model of the ICG-supplied sub-surface target can be created by processor 106 and displayed, in enhanced colors (e.g. green), on the 3D display, as described in Figure 25. In this example, the ICG injection serves several purposes. It helps visualize the kidney's blood supply, thus, reducing blood loss during surgery. It allows the surgeon to clearly see the difference between cancerous and healthy tissue. Hence, the surgeon leaves cancerous tissue behind and spare more healthy tissue. After a tumor is removed and the kidney has been repaired, ICG allows the surgeon to see restored blood flow to the kidney. The sub-surface Q3D model obtained in fluorescent mode can be combined with the surface Q3D model obtained when shinning the surgical scene with white light. To achieve this, it will be appreciated that certain surface contours, or other fiducial points, of the anatomical image of the fluorescent image typically are also visible in the non-fluorescence image. For example, especially portions of the anatomical structure in which the die is not as concentrated may be visible in both images. Similar to the techniques described above, a Q3D model of the anatomical structure can be aligned with the fluorescent image to thereby provide an indication of a relationship between the sub-surface target structure and visible surface contours encompassed within a FOVe of a Q3D endoscope and captured within a corresponding Q3D model. Sub-surface target 3204 visible only in a fluorescent view. The processes presented in Figures 30 and 31 can be used to align the non-fluorescent Q3D model with the fluorescent Q3D model of the sub-surface target. More specifically, surface contour fiducials that are visible in the fluorescent view are aligned with corresponding surface contours in the Q3D model. The fluorescent model is geometrically transformed, e.g. rotated and translated, until alignment of matching surface contours is achieved generally as described with reference to the flow diagram of Figure 30. Dye other than ICG can be used to visualize other anatomical targets. Imaging systems other than the *da Vinci* Si or *da Vinci* Xi Surgical Systems, or the *FireFly* Fluorescent Imaging System, can be equally employed to achieve similar results.

The foregoing description and drawings of embodiments in accordance with the present invention are merely illustrative of the principles of the invention. For example, while certain alignment techniques were presented above, those skilled in the art can use different alignment algorithms to achieve equivalent results. Those skilled in the art would also know how to use other types of Q3D endoscopes, such as those based on time-of-flight imaging sensors, in order to achieve equivalent results. Therefore, it will be understood that various modifications can be made to the embodiments .

**Claims**

1.  A system for producing an image of a surgical scene comprising:

a quantitative three-dimensional, Q3D, endoscope (210) having an imaging sensor array (101), comprising a plurality of imaging sensors ($S_{11}$-$S_{33}$) wherein each imaging sensor includes a corresponding lens stack (186) and a two-dimensional arrangement of pixels including at least two pixels in each dimension arranged in a focal plane (188) of the lens stack (186), wherein each imaging sensor ($S_{11}$-$S_{33}$) and each corresponding focal plane (188) occupies a region of the imaging sensor array (101) different from regions of the imaging sensor array (101) occupied by other imaging sensors ($S_{11}$, $S_{12}$, $S_{13}$) and other corresponding focal planes (188), and wherein at least three of the imaging sensors ($S_{11}$-$S_{33}$) of the imaging sensor array (101) are disposed to have coplanar fields of view ($FOV_1$, $FOV_2$, $FOV_3$) that overlap, wherein the corresponding lens stack (186) of each imaging sensor creates a separate optical channel that resolves an image of a scene within a surgical field of view onto a corresponding arrangement of pixels disposed in a focal plane (188) of the corresponding lens stack (186); at least one processor (106) configured to:

determine a Q3D model (2806) of an external surface contour (2804) of a tissue structure (2602) imaged by three or more different imaging sensors ($S_{11}$-$S_{33}$) of the Q3D endoscope (210); input a 3D visual representation (2601) of the tissue structure (2602);

determine a geometric transformation that aligns an external surface contour (2608) of the 3D visual representation (2601) of the tissue structure (2602) with the Q3D model (2806) of the external surface contour (2804) of said tissue structure (2602); and

produce, based at least in part upon said geometric transformation, a visual output representing the combined Q3D model (2806) of the external surface contour (2804) of the tissue structure (2602) and a sub-surface (2606) of the 3D visual representation (2601) of the tissue structure (2602).

2. The system of claim 1,
   wherein the 3D visual representation (2601) can be one of the following: MRI, CT, PET, ultrasound or fluorescent images.

3. The system of claim 1, wherein the at least one processor (106) is further configured to:

   identify multiple fiducial points on an external surface contour (2804) of the tissue structure (2602) represented in the 3D visual representation (2601);
   identify substantially the same multiple fiducial points of the tissue structure represented within the Q3D model (2806) ; and
   apply a geometric transformation to the 3D visual representation (2601) of the tissue structure (2602) to align the identified fiducial points in the 3D visual representation (2601) with the identified fiducial points in the Q3D model (2806) representation of the tissue structure (2602).

4. The system of claim 1,
   wherein a sub-surface target tissue structure (2606) is visible as ghost image within the visual output.

5. The system of claim 1,
   wherein the at least one processor (106) is further configured to:

   identify a view of a sub-surface target structure (2606) within the aligned 3D visual representation (2601);
   wherein producing a visual output representing the combined Q3D model (2806) of the external surface contour (2804) of the tissue structure and the 3D visual representation of the sub-surface (2606) of tissue structure includes producing an output that includes the identified view of the sub-surface target structure (2606).

6. The system of claim 5,
   wherein the view includes a slice (2604) of the 3D visual representation (2601).

7. The system of claim 1,
   wherein the Q3D model display information includes a display of (x, y, z) coordinates in reference to details visible in the image.

8. The system of claim 1,
   wherein the Q3D model display information includes a display of information indicating distance between points on an external surface of the tissue structure (2602) from the Q3D endoscope (210).

9. A method producing an image of a surgical scene comprising:

imaging a scene of interest within a surgical field of view by using a quantitative three-dimensional, Q3D, endoscope (210) having an imaging sensor array (101), comprising a plurality of imaging sensors ($S_{11}$-$S_{33}$) wherein each imaging sensor includes a corresponding lens stack (186) and a two-dimensional arrangement of pixels (800) including at least two pixels in each dimension arranged in a focal plane (188) of the lens stack (186), wherein each imaging sensor ($S_{11}$-$S_{33}$) and each corresponding focal plane (188) occupies a region of the imaging sensor array (101) different from regions of the imaging sensor array (101) occupied by other imaging sensors ($S_{11}$, $S_{12}$, $S_{13}$) and other corresponding focal planes (188), and wherein at least three of the imaging sensors ($S_{11}$-$S_{33}$) of the imaging sensor array (101) are disposed to have coplanar fields of view ($FOV_1$, $FOV_2$, $FOV_3$) that overlap, wherein the corresponding lens stack (186) of each imaging sensor creates a separate optical channel that resolves an image of a scene within a surgical field of view onto a corresponding arrangement of pixels disposed in a focal plane (188) of the corresponding lens stack (186);
determining a Q3D model (2806) of an external surface contour (2804) of a tissue structure (2602) imaged by three or more different imaging sensors ($S_{11}$-$S_{33}$) of the Q3D endoscope (210);
inputting a 3D visual representation (2601) of the tissue structure (2602);
determining a geometric transformation that aligns an external surface contour (2608) of the 3D visual representation (2601) of the tissue structure (2602) with the Q3D model (2806) of the external surface contour (2804) of said tissue structure (2602); and
producing, based at least in part upon said geometric transformation, a visual output representing the combined Q3D model (2806) of the external surface contour (2804) of the tissue structure (2602) and a sub-surface (2606) of the 3D visual representation (2601) of the tissue structure (2602).

10. The method of claim 9,
wherein producing the combined Q3D model (2806) and 3D visual representation (2601) is based, at least in part, upon:

identifying multiple fiducial points on an external surface contour (2804) of the tissue structure represented in the 3D visual representation (2601);
identifying substantially the same multiple fiducial points within the Q3D model (2806);
applying a geometric transformation to the 3D visual representation (2601) of the tissue structure (2602) to align the identified fiducial points in the 3D visual representation (2601) of the tissue structure (2602) with the identified fiducial points in the Q3D model (2806) representation of the tissue structure (2602).

11. The method of claim 9,
wherein the 3D visual representation (2601) can be one of the following: MRI, CT, PET, ultrasound or fluorescent images.

12. The method of claim 9,
wherein a sub-surface target tissue structure (2606) is visible as ghost image within the visual output.

13. The method of claim 9 further including:

identifying a view of a sub-surface target structure (2602) within the aligned 3D visual representation (2601);
wherein producing a visual output representing the combined Q3D model (2806) of the external surface contour (2804) of the tissue structure (2602) and a sub-surface (2606) of the 3D visual representation (2601) of the tissue structure (2602) includes producing an output that includes the identified view of the sub-surface target structure (2606).

14. The method of claim 13,
wherein the view includes a slice (2604) of the 3D visual representation (2601), or
wherein the Q3D model (2806) display information includes a display of (x, y, z) coordinates in reference to details visible in the image.

15. The method of claim 9,
wherein the Q3D model display information includes a display of information indicating distance between points on an outer surface of the tissue structure (2602) from the Q3D endoscope (210).

**Patentansprüche**

1. Ein System zur Herstellung eines Bilds einer chirurgischen Szene, das Folgendes beinhaltet:

    ein quantitatives dreidimensionales (Q3D) Endoskop (210) mit einem Bildgebungssensorarray (101), das eine Vielzahl von Bildgebungssensoren ($S_{11}$-$S_{33}$) beinhaltet, wobei jeder Bildgebungssensor eine entsprechende Linsenbaugruppe (186) und eine zweidimensionale Anordnung von Pixeln umfasst, die in jeder Dimension mindestens zwei Pixel umfasst, die in einer fokalen Ebene (188) der Linsenbaugruppe (186) angeordnet sind, wobei jeder Bildgebungssensor ($S_{11}$-$S_{33}$) und jede entsprechende fokale Ebene (188) eine Region des Bildgebungssensorarrays (101) belegt, die sich von Regionen des Bildgebungssensorarrays (101) unterscheidet, die von anderen Bildgebungssensoren ($S_{11}$, $S_{12}$, $S_{13}$) und anderen entsprechenden fokalen Ebenen (188) belegt sind, und wobei mindestens drei der Bildgebungssensoren ($S_{11}$-$S_{33}$) des Bildgebungssensorarrays (101) so eingerichtet sind, dass sie überlappende koplanare Sehfelder ($FOV_1$, $FOV_2$, $FOV_3$) aufweisen, wobei die entsprechende Linsenbaugruppe (186) jedes Bildgebungssensors einen separaten optischen Kanal schafft, der ein Bild einer Szene innerhalb eines chirurgischen Sehfelds auf eine entsprechende Anordnung von Pixeln auflöst, die in einer fokalen Ebene (188) der entsprechenden Linsenbaugruppe (186) eingerichtet sind;
    mindestens einen Prozessor (106), der zu Folgendem ausgelegt ist:

        Bestimmen eines Q3D-Modells (2806) einer externen Oberflächenkontur (2804) einer Gewebestruktur (2602), die durch drei oder mehr verschiedene Bildgebungssensoren ($S_{11}$-$S_{33}$) des Q3D-Endoskops (210) abgebildet wird;
        Eingeben einer visuellen 3D-Darstellung (2601) der Gewebestruktur (2602);
        Bestimmen einer geometrischen Transformation, die eine externe Oberflächenkontur (2608) der visuellen 3D-Darstellung (2601) der Gewebestruktur (2602) mit dem Q3D-Modell (2806) der externen Oberflächenkontur (2804) der Gewebestruktur (2602) ausrichtet; und
        Herstellen, mindestens teilweise auf der Grundlage der geometrischen Transformation, einer visuellen Ausgabe, die das kombinierte Q3D-Modell (2806) der externen Oberflächenkontur (2804) der Gewebestruktur (2602) und eine Teiloberfläche (2606) der visuellen 3D-Darstellung (2601) der Gewebestruktur (2602) darstellt.

2. System nach Anspruch 1,
    wobei die visuelle 3D-Darstellung (2601) eines von den Folgenden sein kann: MRT-, CT-, PET-, Ultraschall- oder Fluoreszenzbilder.

3. System nach Anspruch 1, wobei der mindestens eine Prozessor (106) ferner zu Folgendem ausgelegt ist:

        Identifizieren mehrerer Lokalisationspunkte auf einer externen Oberflächenkontur (2804) der Gewebestruktur (2602), die in der visuellen 3D-Darstellung (2601) dargestellt sind;
        Identifizieren im Wesentlichen der gleichen mehreren Lokalisationspunkte der Gewebestruktur, die innerhalb des Q3D-Modells (2806) dargestellt sind, und
        Anwenden einer geometrischen Transformation auf die visuelle 3D-Darstellung (2601) der Gewebestruktur (2602), um die identifizierten Lokalisationspunkte in der visuellen 3D-Darstellung (2601) mit den identifizierten Lokalisationspunkten in der Q3D-Modell(2806)-Darstellung der Gewebestruktur (2602) auszurichten.

4. System nach Anspruch 1,
    wobei eine Teiloberflächen-Zielgewebestruktur (2606) als Geisterbild innerhalb der visuellen Ausgabe sichtbar ist.

5. System nach Anspruch 1,
    wobei der mindestens eine Prozessor (106) ferner zu Folgendem ausgelegt ist:

        Identifizieren einer Ansicht einer Teiloberflächen-Zielstruktur (2606) innerhalb der ausgerichteten visuellen 3D-Darstellung (2601);
        wobei das Herstellen einer visuellen Ausgabe, die das kombinierte Q3D-Modell (2806) der externen Oberflächenkontur (2804) der Gewebestruktur und die visuelle 3D-Darstellung der Teiloberfläche (2606) der Gewebestruktur darstellt, das Herstellen einer Ausgabe umfasst, die die identifizierte Ansicht der Teiloberflächen-Zielstruktur (2606) umfasst.

6. System nach Anspruch 5,

wobei die Ansicht ein Schichtbild (2604) der visuellen 3D-Darstellung (2601) umfasst.

7. System nach Anspruch 1,
   wobei die Q3D-Modell-Anzeigeinformationen eine Anzeige von (x,y,z)-Koordinaten, die sich auf in dem Bild sichtbare Einzelheiten beziehen, umfassen.

8. System nach Anspruch 1,
   wobei die Q3D-Modell-Anzeigeinformationen eine Anzeige von Informationen umfassen, die den Abstand zwischen Punkten auf einer externen Oberfläche der Gewebestruktur (2602) von dem Q3D-Endoskop (210) angeben.

9. Ein Verfahren zum Herstellen eines Bilds einer chirurgischen Szene, das Folgendes beinhaltet:

   Abbilden einer Szene von Interesse innerhalb eines chirurgischen Sehfelds durch Verwenden eines quantitativen dreidimensionalen (Q3D) Endoskops (210) mit einem Bildgebungssensorarray (101), das eine Vielzahl von Bildgebungssensoren ($S_{11}$-$S_{33}$) beinhaltet, wobei jeder Bildgebungssensor eine entsprechende Linsenbaugruppe (186) und eine zweidimensionale Anordnung von Pixeln (800) umfasst, die mindestens zwei Pixel in jeder Dimension umfasst, die in einer fokalen Ebene (188) der Linsenbaugruppe (186) angeordnet sind, wobei jeder Bildgebungssensor ($S_{11}$-$S_{33}$) und jede entsprechende fokale Ebene (188) eine Region des Bildgebungssensorarrays (101) belegt, die sich von Regionen des Bildgebungssensorarrays (101) unterscheidet, die von anderen Bildgebungssensoren ($S_{11}$, $S_{12}$, $S_{13}$) und anderen entsprechenden fokalen Ebenen (188) belegt sind, und wobei mindestens drei der Bildgebungssensoren ($S_{11}$-$S_{33}$) des Bildgebungssensorarrays (101) so eingerichtet sind, dass sie überlappende koplanare Sehfelder ($FOV_1$, $FOV_2$, $FOV_3$) aufweisen, wobei die entsprechende Linsenbaugruppe (186) jedes Bildgebungssensors einen separaten optischen Kanal schafft, der ein Bild einer Szene innerhalb eines chirurgischen Sehfelds auf eine entsprechende Anordnung von Pixeln auflöst, die in einer fokalen Ebene (188) der entsprechenden Linsenbaugruppe (186) eingerichtet sind;
   Bestimmen eines Q3D-Modells (2806) einer externen Oberflächenkontur (2804) einer Gewebestruktur (2602), die durch drei oder mehr verschiedene Bildgebungssensoren ($S_{11}$-$S_{33}$) des Q3D-Endoskops (210) abgebildet wird;
   Eingeben einer visuellen 3D-Darstellung (2601) der Gewebestruktur (2602);
   Bestimmen einer geometrischen Transformation, die eine externe Oberflächenkontur (2608) der visuellen 3D-Darstellung (2601) der Gewebestruktur (2602) mit dem Q3D-Modell (2806) der externen Oberflächenkontur (2804) der Gewebestruktur (2602) ausrichtet; und
   Herstellen, mindestens teilweise auf der Grundlage der geometrischen Transformation, einer visuellen Ausgabe, die das kombinierte Q3D-Modell (2806) der externen Oberflächenkontur (2804) der Gewebestruktur (2602) und eine Teiloberfläche (2606) der visuellen 3D-Darstellung (2601) der Gewebestruktur (2602) darstellt.

10. Verfahren nach Anspruch 9,
    wobei das Herstellen des kombinierten Q3D-Modells (2806) und der visuellen 3D-Darstellung (2601) mindestens teilweise auf Folgendem basiert:

    Identifizieren mehrerer Lokalisationspunkte auf einer externen Oberflächenkontur (2804) der Gewebestruktur, die in der visuellen 3D-Darstellung (2601) dargestellt sind;
    Identifizieren im Wesentlichen der gleichen mehreren Lokalisationspunkte innerhalb des Q3D-Modells (2806);
    Anwenden einer geometrischen Transformation auf die visuelle 3D-Darstellung (2601) der Gewebestruktur (2602), um die identifizierten Lokalisationspunkte in der visuellen 3D-Darstellung (2601) der Gewebestruktur (2602) mit den identifizierten Lokalisationspunkten in der Q3D-Modell(2806)-Darstellung der Gewebestruktur (2602) auszurichten.

11. Verfahren nach Anspruch 9,
    wobei die visuelle 3D-Darstellung (2601) eines von den Folgenden sein kann: MRT-, CT-, PET-, Ultraschall- oder Fluoreszenzbilder.

12. Verfahren nach Anspruch 9,
    wobei eine Teiloberflächen-Zielgewebestruktur (2606) als Geisterbild innerhalb der visuellen Ausgabe sichtbar ist.

13. Verfahren nach Anspruch 9, das ferner Folgendes umfasst:

    Identifizieren einer Ansicht einer Teiloberflächen-Zielstruktur (2602) innerhalb der ausgerichteten visuellen 3D-Darstellung (2601);
wobei das Herstellen einer visuellen Ausgabe, die das kombinierte Q3D-Modell (2806) der externen Oberflächenkontur (2804) der Gewebestruktur (2602) und eine Teiloberfläche (2606) der visuellen 3D-Darstellung (2601) der Gewebestruktur (2602) darstellt, das Herstellen einer Ausgabe umfasst, die die identifizierte Ansicht der Teiloberflächen-Zielstruktur (2606) umfasst.

14. Verfahren nach Anspruch 13,
wobei die Ansicht ein Schichtbild (2604) der visuellen 3D-Darstellung (2601) umfasst oder
wobei die Q3D-Modell(2806)-Anzeigeinformationen eine Anzeige von (x,y,z)-Koordinaten, die sich auf in dem Bild sichtbare Einzelheiten beziehen, umfassen.

15. Verfahren nach Anspruch 9,
wobei die Q3D-Modell-Anzeigeinformationen eine Anzeige von Informationen umfassen, die den Abstand zwischen Punkten auf einer äußeren Oberfläche der Gewebestruktur (2602) von dem Q3D-Endoskop (210) angeben.

**Revendications**

1. Système destiné à produire une image d'une scène chirurgicale comprenant :

   un endoscope tridimensionnel quantitatif, Q3D, (210) ayant un réseau de capteurs d'imagerie (101) comprenant une pluralité de capteurs d'imagerie ($S_{11}$ à $S_{33}$) dans lequel chaque capteur d'imagerie inclut un empilement de lentilles correspondant (186) et un agencement bidimensionnel de pixels incluant au moins deux pixels dans chaque dimension agencés dans un plan focal (188) de l'empilement de lentilles (186), dans lequel chaque capteur d'imagerie ($S_{11}$ à $S_{33}$) et chaque plan focal correspondant (188) occupent une région du réseau de capteurs d'imagerie (101) différente des régions du réseau de capteurs d'imagerie (101) occupées par d'autres capteurs d'imagerie ($S_{11}$, $S_{12}$, $S_{13}$) et d'autres plans focaux correspondants (188), et dans lequel au moins trois des capteurs d'imagerie ($S_{11}$ à $S_{33}$) du réseau de capteurs d'imagerie (101) sont disposés pour avoir des champs de vision coplanaires ($FOV_1$, $FOV_2$, $FOV_3$) qui se chevauchent, dans lequel l'empilement de lentilles correspondant (186) de chaque capteur d'imagerie crée un canal optique distinct qui résout une image d'une scène au sein d'un champ de vision chirurgical sur un agencement correspondant de pixels disposés dans un plan focal (188) de l'empilement de lentilles correspondant (186) ;
   au moins un processeur (106) configuré pour :

      déterminer un modèle Q3D (2806) d'un contour de surface externe (2804) d'une structure de tissu (2602) imagée par trois capteurs d'imagerie différents ($S_{11}$ à $S_{33}$) ou plus de l'endoscope Q3D (210) ;
      entrer une représentation visuelle 3D (2601) de la structure de tissu (2602) ;
      déterminer une transformation géométrique qui aligne un contour de surface externe (2608) de la représentation visuelle 3D (2601) de la structure de tissu (2602) avec le modèle Q3D (2806) du contour de surface externe (2804) de ladite structure de tissu (2602) ; et
      produire, sur la base au moins en partie de ladite transformation géométrique, une sortie visuelle représentant le modèle Q3D combiné (2806) du contour de surface externe (2804) de la structure de tissu (2602) et une sous-surface (2606) de la représentation visuelle 3D (2601) de la structure de tissu (2602).

2. Système selon la revendication 1,
dans lequel la représentation visuelle 3D (2601) peut être l'une des suivantes : des images IRM, CT, PET, échographiques ou fluorescentes.

3. Système selon la revendication 1, dans lequel l'au moins un processeur (106) est configuré en outre pour :

   identifier des points fiduciels multiples sur un contour de surface externe (2804) de la structure de tissu (2602) représentée dans la représentation visuelle 3D (2601) ;
   identifier substantiellement les mêmes points fiduciels multiples de la structure de tissu représentée au sein du modèle Q3D (2806) et
   appliquer une transformation géométrique à la représentation visuelle 3D (2601) de la structure de tissu (2602) pour aligner les points fiduciels identifiés dans la représentation visuelle 3D (2601) avec les points fiduciels identifiés dans la représentation de modèle Q3D (2806) de la structure de tissu (2602).

**4.** Système selon la revendication 1,
dans lequel une structure de tissu cible de sous-surface (2606) est visible comme une image fantôme au sein de la sortie visuelle.

**5.** Système selon la revendication 1,
dans lequel l'au moins un processeur (106) est configuré en outre pour :

identifier une vue d'une structure cible de sous-surface (2606) au sein de la représentation visuelle 3D alignée (2601) ;
dans lequel la production d'une sortie visuelle représentant le module Q3D combiné (2806) du contour de surface externe (2804) de la structure de tissu et la représentation visuelle 3D de la sous-surface (2606) de la structure de tissu inclut la production d'une sortie qui inclut la vue identifiée de la structure cible de la sous-surface (2606).

**6.** Système selon la revendication 5,
dans lequel la vue inclut une tranche (2604) de la représentation visuelle 3D (2601).

**7.** Système selon la revendication 1,
dans lequel les informations d'affichage de modèle Q3D incluent un affichage (3300) de coordonnées (x, y, z) en référence à des détails visibles dans l'image.

**8.** Système selon la revendication 1,
dans lequel les informations d'affichage de modèle Q3D incluent un affichage d'informations indiquant la distance entre des points sur une surface externe de la structure de tissu (2602) de l'endoscope Q3D (210).

**9.** Procédé produisant une image d'une scène chirurgicale comprenant :

l'imagerie d'une scène d'intérêt au sein d'un champ de vision chirurgical en utilisant un endoscope tridimensionnel quantitatif, Q3D, (210) ayant un réseau de capteurs d'imagerie (101), comprenant une pluralité de capteurs d'imagerie ($S_{11}$ à $S_{33}$) dans lequel chaque capteur d'imagerie inclut un empilement de lentilles correspondant (186) et un agencement bidimensionnel de pixels incluant au moins deux pixels (800) dans chaque dimension agencés dans un plan focal (188) de l'empilement de lentilles (186), dans lequel chaque capteur d'imagerie ($S_{11}$ à $S_{33}$) et chaque plan focal correspondant (188) occupent une région du réseau de capteurs d'imagerie (101) différente des régions du réseau de capteurs d'imagerie (101) occupées par d'autres capteurs d'imagerie ($S_{11}$, $S_{12}$, $S_{13}$) et d'autres plans focaux correspondants (188), et dans lequel au moins trois des capteurs d'imagerie ($S_{11}$ à $S_{33}$) du réseau de capteurs d'imagerie (101) sont disposés pour avoir des champs de vision coplanaires ($FOV_1$, $FOV_2$, $FOV_3$) qui se chevauchent, dans lequel l'empilement de lentilles correspondant (186) de chaque capteur d'imagerie crée un canal optique distinct qui résout une image d'une scène au sein d'un champ de vision chirurgical sur un agencement correspondant de pixels disposés dans un plan focal (188) de l'empilement de lentilles correspondant (186) ;
la détermination d'un modèle Q3D (2806) d'un contour de surface externe (2804) d'une structure de tissu (2602) imagée par trois capteurs d'imagerie différents ($S_{11}$ à $S_{33}$) ou plus de l'endoscope Q3D (210) ;
l'entrée d'une représentation visuelle 3D (2601) de la structure de tissu (2602) ;
la détermination d'une transformation géométrique qui aligne un contour de surface externe (2608) de la représentation visuelle 3D (2601) de la structure de tissu (2602) avec le modèle Q3D (2806) du contour de surface externe (2804) de ladite structure de tissu (2602) ; et
la production, sur la base au moins en partie de ladite transformation géométrique, d'une sortie visuelle représentant le modèle Q3D combiné (2806) du contour de surface externe (2804) de la structure de tissu (2602) et une sous-surface (2606) de la représentation visuelle 3D (2601) de la structure de tissu (2602).

**10.** Procédé selon la revendication 9,
dans lequel la production du modèle Q3D combiné (2806) et de la représentation visuelle 3D (2601) est basée, au moins en partie, sur :

l'identification de points fiduciels multiples sur un contour de surface externe (2804) de la structure de tissu (2602) représentée dans la représentation visuelle 3D (2601) ;
l'identification substantiellement des mêmes points fiduciels multiples au sein du modèle Q3D (2806) ;
l'application d'une transformation géométrique à la représentation visuelle 3D (2601) de la structure de tissu (2602) pour aligner les points fiduciels identifiés dans la représentation visuelle 3D (2601) de la structure de tissu (2602) avec les points fiduciels identifiés dans la représentation du modèle Q3D (2806) de la structure de tissu (2602).

11. Procédé selon la revendication 9,
dans lequel la représentation visuelle 3D (2601) peut être l'une des suivantes : des images IRM, CT, PET, échographiques ou fluorescentes.

12. Procédé selon la revendication 9,
dans lequel une structure de tissu cible de sous-surface (2606) est visible comme une image fantôme au sein de la sortie visuelle.

13. Procédé selon la revendication 9 incluant en outre :

l'identification d'une vue d'une structure cible de sous-surface (2602) au sein de la représentation visuelle 3D alignée (2601) ;
dans lequel la production d'une sortie visuelle représentant le module Q3D combiné (2806) du contour de surface externe (2804) de la structure de tissu et une sous-surface (2606) de représentation visuelle 3D (2601) de la structure de tissu (2602) inclut la production d'une sortie qui inclut la vue identifiée de la structure cible de sous-surface (2606).

14. Procédé selon la revendication 13,
dans lequel la vue inclut une tranche (2604) de la représentation visuelle 3D (2601), ou
dans lequel les informations d'affichage de modèle Q3D incluent un affichage (2806) de coordonnées (x, y, z) en référence à des détails visibles dans l'image.

15. Procédé selon la revendication 9,
dans lequel les informations d'affichage de modèle Q3D incluent un affichage d'informations indiquant la distance entre des points sur une surface externe de la structure de tissu (2602) de l'endoscope Q3D (210).

*FIG. 1*

(PRIOR ART)

*FIG. 2*

(PRIOR ART)

## FIG. 3
(PRIOR ART)

*FIG. 4*

EP 3 122 232 B1

*FIG. 5*

*FIG. 6*

*FIG. 7A*

*FIG. 7B*

*FIG. 8*

Acquire Video Data
From Imaging
Sensor(s) sij
401

Start Scanning Imaging
Volume And Assign Initial
Target Value
402

Compute (x, y, z,) Of
Initial Target
403

Refine
Computation
404

Has The Entire Imaging
Volume Of Interest Been
Scanned?
405

Store 3D Model Data
For Further Review/
Manipulation
407

Assemble 3D Model Of
Imaging Volume Of
Interest
406

Display Quantitative
3D View
408

*FIG. 9*

FIG. 10

*FIG. 11*

*FIG. 12*

*FIG. 13*

*FIG. 14*

(Best Shape Match)

*FIG. 15*

FIG. 16

*FIG. 17*

N Pixels

N Pixels

$T(x_{11}, y_{11})$

$*$

$S_{11}$

$[n_{x1}, n_{y1}]$

x

y   z

N Pixels

N Pixels

$T(x_{12}, y_{12})$

$*$

$S_{12}$

$[n_{x2}, n_{y2}]$

x

y   z

N Pixels

N Pixels

$T(x_{13}, y_{13})$

$*$

$S_{13}$

$[n_{x3}, n_{y3}]$

x

y   z

*FIG. 18*

*FIG. 19*

2000

Receive User Input to
Select Multiple Objects
2002

↓ Yes

Display Menu
2004

Receive
User Input To Display
Distance?
2006

No

Time Out?
2010

End

Yes

Display Distance
Within User FOV
2008

Receive
User Input To
Enter Proximity Alarm
2012

No

Time Out?
2020

End

Yes

Monitor Distance
2014

Proximity
Threshold Crossed?
2016

No

Yes

Activate Alarm
2018

*FIG. 20*

2102

2104

Display Distance
2106

Set Proximity Alarm
2108

2110

XXXX

Enter Distance

*FIG. 21*

*FIG. 22A*

EP 3 122 232 B1

FIG. 22B

EP 3 122 232 B1

Receive User Input toSelect
Multiple Object
2302

Display Menu
2304

Receive
User Input To
Rotate?
2306

Time Out?
2310

End

Rotate
2308

FIG. 23

2402

2404

Rotate Left
2406

Rotate Right
2408

*FIG. 24*

FIG. 25

*FIG. 26*

2702

Contrast-enhancing Mass
2706

2704

G2    G3

2704  Trachea

T1

2708

*FIG. 27A*

*FIG. 27B*

FIG. 28

FIG. 29

3000

Select External
Fiducial Points
in Q3D
— 3002

Select Same
External
Fiducial Points
in 3D
— 3004

Apply Geometrical
Transformations to
3D MRI / or
Representation until
Common Fiducial Points
Match With Those
of Q3D Model
— 3006

3008

Best Match
of Common Fiducial Points
Achieved?

No

Yes

Q3D-MRI / CT
Combination Model
in Alignment
— 3010

*FIG. 30*

3200

```
┌─────────────────────┐
│  Identify View Of Target  │
│ Structure That Aligns With │ ∼ 3102
│  Aligned Surface View Of  │
│        Contour        │
└─────────────────────┘
           │
           ▼
┌─────────────────────┐
│    Combine Q3D Model    │
│  With The Identified View  │ ∼ 3104
│   Of The Target Structure  │
│ Within A 3D Representation  │
└─────────────────────┘
           │
           ▼
┌─────────────────────┐
│                     │
│      Display The      │ ∼ 3106
│      Combination      │
│                     │
└─────────────────────┘
```

## FIG. 31

3200

3202

FOV_e

101C

FOV_e

3204

FIG. 32

FIG. 33A

FIG. 33B

FIG. 33C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61971749
- US 62096515
- US 6323942 B
- US 8262559 B
- US 20120190923
- US 8514491 B
- US 20130070060
- US 8514491 B2
- US 20130070060 A1

- US 6650927 B
- US 2015049167 A
- US 20120020547 A
- US 8514 A
- US 491 A
- US 20130070060 A
- US 4562463 A, Lipton
- US 6008839 A, Nagele

### Non-patent literature cited in the description

- **C. SCHMALZ et al.** An endoscopic 3D scanner based on structured light. *Medical Image Analysis,* 2012, vol. 16, 1063-1072